# EUROPEAN PATENT APPLICATION

(11) **EP 4 730 323 A1**
(43) Date of publication of application: **22.04.2026**
(21) Application number: 24853132.9
(22) Date of filing: 31.01.2024
(51) Int. Cl.: G10L 15/22

(54) **INTELLIGENT CONTROL METHOD AND SYSTEM**

(30) Priority: 15.08.2023 CN 202311028614; 15.08.2023 CN 202311028926; 29.12.2023 CN 202311863887
(71) Applicant: Shanghai United Imaging Healthcare Co., Ltd., Shanghai 201807 (CN)
(72) Inventor: HAN, Chenghang, Shanghai 201807 (CN); GU, Jie, Shanghai 201807 (CN); ZHOU, Xintong, Shanghai 201807 (CN); ZHU, Mengqi, Shanghai 201807 (CN)
(74) Representative: Wang, Bo
(86) International application number: PCT/CN2024/075034
(87) International publication number: WO 2025/035715

(57) **Abstract**

A method and a system for intelligent control are provided. The method includes: obtaining a first voice signal (1502), the first voice signal (1502) being generated by a first voice device (940) through detecting a first user voice; obtaining a voice recognition result (1504) by performing a voice recognition on the first voice signal (1502); determining, based on the voice recognition result (1504), whether the first user voice contains a control instruction (1506) for a target device; and in response to determining that the first user voice contains the control instruction (1506) for the target device, controlling the target device based on the control instruction (1506).

## Description

### CROSS REFERENCE TO RELATED APPLICATIONS

This application claims priority to Chinese patent application No. 202311028614.6 filed on August 15, 2023, Chinese patent application No. 202311028926.7 filed on August 15, 2023, and Chinese patent application No. 202311863887.2 filled on December 29, 2023, the entire contents of which are incorporated herein by reference.

### TECHNICAL FIELD

The present disclosure generally relates to a field of intelligent control, and in particular, to methods and systems for intelligent control used in medical field.

### BACKGROUND

In medical scenarios (e.g., a digital subtraction angiography (DSA) examination, an X-ray scanning in hospital), doctors, technicians, etc. often need to manually control devices in an examination room, such as a display device, an imaging device, etc. However, a manual control is time-consuming and laborious, which leads to a low execution efficiency of a medical procedure and affects a normal progress of a medical procedure due to mis-operation.

Therefore, methods and systems for intelligent control are provided to assist the doctor in controlling devices in the examination room accurately and automatically through voice, thereby improving execution efficiency and accuracy of the medical procedure.

### SUMMARY

One or more embodiments of the present disclosure provide a method for intelligent control. The method may include obtaining a first voice signal. The first voice signal is generated by a first voice device through detecting a first user voice. The method may include obtaining a voice recognition result by performing a voice recognition on the first voice signal. The method may include determining, based on the voice recognition result, whether the first user voice contains a control instruction for a target device. The method may further include, in response to determining that the first user voice contains the control instruction for the target device, controlling the target device based on the control instruction.

One or more embodiments of the present disclosure provide a system for intelligent control including an obtaining module, a recognition module, a determination module, and a control module. The obtaining module is configured to obtain a first voice signal, the first voice signal being generated by a first voice device through detecting a first user voice. The recognition module is configured to obtain a voice recognition result by performing a voice recognition on the first voice signal. The determination module is configured determine, based on the voice recognition result, whether the first user voice contains a control instruction for a target device. The control module is configured to, in response to determining that the first user voice contains the control instruction for the target device, control the target device based on the control instruction.

One or more embodiments of the present disclosure provide a system for intelligent control. The system may include a storage device and a processor. The storage device may store computer instructions; the processor is connected to the storage device. When the computer instructions are executed , the processor makes the system to perform the following operations: obtaining a first voice signal, the first voice signal being generated by a first voice device through detecting a first user voice; obtaining a voice recognition result by performing a voice recognition on the first voice signal; determining, based on the voice recognition result, whether the first user voice contains a control instruction for a target device; and in response to determining that the first user voice contains the control instruction for the target device, controlling the target device based on the control instruction.

One or more embodiments of the present disclosure provide a computer-readable storage medium, the storage medium storing computer instructions, when a computer reads the computer instructions from the storage medium, the computer executes the method for intelligent control.

### BRIEF DESCRIPTION OF THE DRAWINGS

The present disclosure is further described in an illustrative manner by embodiments, which are described in detail with reference to the accompanying drawings. These embodiments are not limiting, and in these embodiments, the same reference numerals denote the same structures, wherein:
FIG. 1A is a schematic diagram illustrating an application scenario for an exemplary intelligent control system according to some embodiments of the present disclosure;
FIG. 1B is a schematic diagram illustrating an exemplary X-ray imaging device according to some embodiments of the present disclosure;
FIG. 1C is a schematic diagram illustrating an exemplary voice device according to some embodiments of the present disclosure;
FIG. 1D is a schematic diagram illustrating an exemplary microphone according to some embodiments of the present disclosure;
FIG. 1E is a schematic diagram illustrating an exemplary speaker according to some embodiments of the present disclosure;
FIG. 2 is a block diagram illustrating an exemplary processing device according to some embodiments of the present disclosure;
FIG. 3 is a flowchart illustrating an exemplary intelligent control process according to some embodiments of the present disclosure;
FIG. 4 is a flowchart illustrating an exemplary voice recognition process according to some embodiments of the present disclosure;
FIG. 5 is a flowchart illustrating an exemplary intelligent control process for a display device according to some embodiments of the present disclosure;
FIG. 6A is a schematic diagram illustrating an exemplary display interface of a display device according to some embodiments of the present disclosure;
FIG. 6B is a schematic diagram illustrating an exemplary updated display interface according to some embodiments of the present disclosure;
FIG. 7 is a flowchart illustrating an exemplary collision detection process according to some embodiments of the present disclosure;
FIG. 8A is a schematic diagram illustrating an exemplary process for controlling motions of one or more components of an X-ray imaging device according to some embodiments of the present disclosure;
FIG. 8B is a schematic diagram illustrating another exemplary process for controlling motions of one or more components of an X-ray imaging device according to some embodiments of the present disclosure;
FIG. 9 is a schematic diagram illustrating an exemplary intelligent control system according to some embodiments of the present disclosure;
FIG. 10 is a schematic diagram illustrating an exemplary intelligent control system according to some embodiments of the present disclosure;
FIG. 11 is a schematic diagram illustrating an exemplary intelligent control system according to some embodiments of the present disclosure;
FIG. 12 is a schematic diagram illustrating an exemplary intelligent control system according to some embodiments of the present disclosure;
FIG. 13 is a schematic diagram illustrating an exemplary intelligent control system according to some embodiments of the present disclosure;
FIG. 14 is a schematic diagram illustrating an exemplary intelligent control system according to some embodiments of the present disclosure;
FIG. 15 is a schematic diagram illustrating an exemplary voice interaction process between a first voice device and a second voice device according to some embodiments of the present disclosure; and
FIG. 16 is a flowchart illustrating an exemplary process for controlling a medical device according to some embodiments of the present disclosure.

### DETAILED DESCRIPTION

The more clearly illustrate the technical solutions of the embodiments of the present disclosure, the accompanying drawings used in the description of the embodiments will be briefly introduced below. Obviously, the accompanying drawings in the following description are merely some examples or embodiments of the present disclosure. For those skilled in the art, without creative effort, the present disclosure may also be applied to other similar scenarios based on these accompanying drawings. Unless obviously obtained from the context or the context illustrates otherwise, the same numeral in the drawings refers to the same structure or operation.

It should be understood that the terms "system," "device," "unit," and/or "module" used herein are a method for distinguishing components, elements, parts, sections, or components of different levels. However, if other words may achieve the same purpose, the words may be replaced by other expressions.

As shown in the present disclosure and the claims, unless the context clearly indicates an exception, the words "a," "an," "one," and/or "the" are not specifically limited to the singular and can also include the plural. Generally, the terms "include" and "comprise" only suggest the inclusion of explicitly identified operations and elements, and these operations and elements do not constitute an exclusive list. The method or device may also include other operations or elements.

The present disclosure uses flowcharts to illustrate the operations performed by the system according to the embodiments of the present disclosure. It should be understood that preceding or following operations are not necessarily performed precisely in sequence. Conversely, various operations may be processed in reverse order or simultaneously. Meanwhile, other operations may be added to these processes, or one or more operations may be removed from these processes.

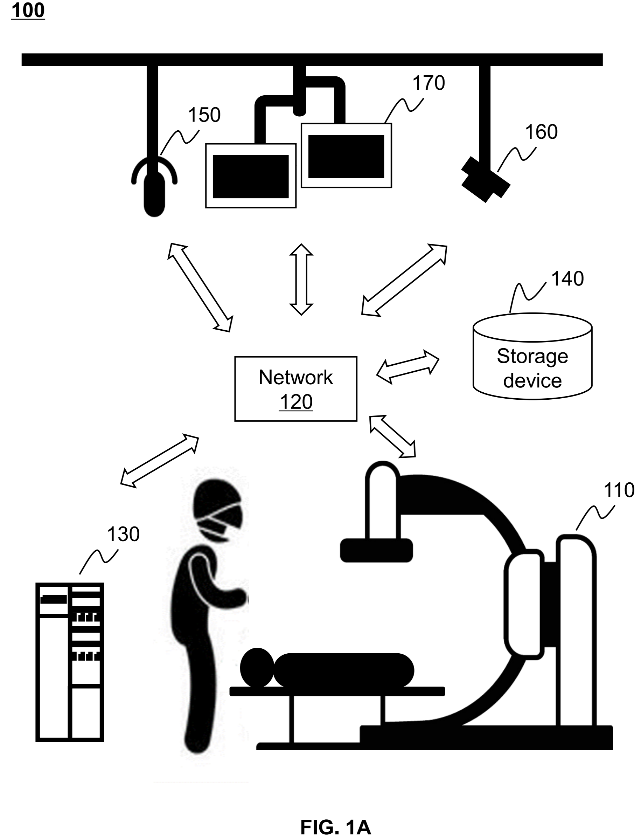

FIG. 1A is a schematic diagram illustrating an application scenario for an exemplary intelligent control system according to some embodiments of the present disclosure.

As shown in FIG. 1A, an intelligent control system 100 includes a medical device 110, a network 120, a processing device 130, a storage device 140, a voice device 150, an image obtaining device 160, and a display device 170. Components in the intelligent control system 100 may be connected in various manners. For example, the medical device 110 and the processing device 130 are connected or communicated via the network 120. As another example, the processing device 130 is directly connected to the medical device 110.

The medical device 110 may be a device for performing a medical procedure, which is installed in an examination room (e.g., an operating room, a scanning room). For example, the medical device 110 includes a medical scanning device for performing a medical scanning procedure (e.g., an X-ray imaging device, an ultrasound scanner, a computed tomography (CT) scanner, a magnetic resonance imaging (MRI) scanner, a positron emission tomography-computed tomography (PET-CT) scanner, an X-ray imaging-MRI scanner, a PET-X-ray imaging scanner, a PET-CT scanner, etc.). As another example, the medical device 110 includes a device for performing surgery, for example, a digital subtraction angiography (DSA) surgery, a radiotherapy surgery, etc. The above medical devices are for illustrative purposes only and are not intended to limit the scope of the present disclosure.

Merely by way of example, the medical device 110 is the X-ray imaging device. The X-ray imaging device may be used to scan a target region of a target object using X-rays and generate a medical image (e.g., a scan image). The target object may include a biological object (e.g., a human body, an animal, etc.), and a non-biological object (e.g., a phantom), etc. In some embodiments, the target region of the target object includes a specific part, organ, and/or tissue of the target object. For example, the target region of the target object includes a head, a chest, a leg, or the like, or any combination thereof, which is not limited herein. In some embodiments, the target region of the target object includes a specific part, organ, and/or tissue of the target object and other organs and/or tissues within a certain range around. The target object may also be referred to as a patient hereinafter.

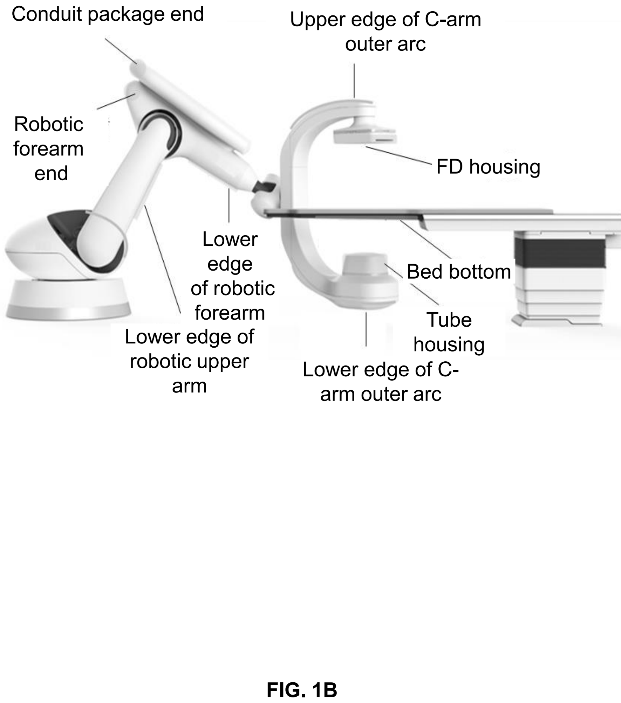

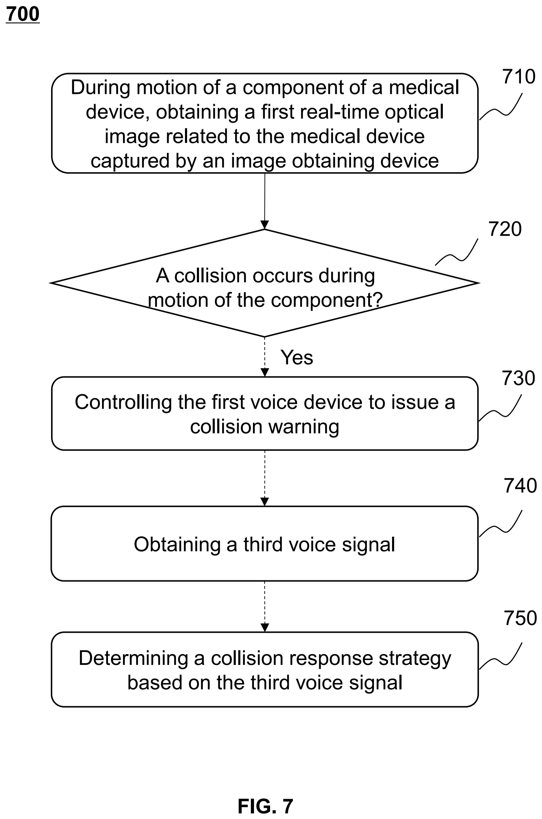

In some embodiments, the X-ray imaging device includes one or more components. For example, the X-ray imaging device is a C-arm X-ray imaging device, which includes a robotic forearm, a robotic upper arm, a C-arm gantry, and a bed plate, etc. In some embodiments, during a motion of one or more components of the X-ray imaging device, to enable a timely collision warning, pressure sensors are installed on the one or more components of the X-ray imaging device. Merely by way of example, FIG. 1B shows the X-ray imaging device. The pressure sensors are installed on parts of the device such as a robotic forearm end, a lower edge of robotic forearm, a lower edge of the robotic upper arm, a conduit package end, an upper edge of C-arm outer arc, a lower edge of C-arm outer arc, a bed bottom, a flat detector (FD) housing, a tube housing, etc. In some embodiments, when a collision occurs at the part where the pressure sensor is installed and a pressure of the collision is greater than a pressure threshold, the X-ray imaging device sends a collision warning to the processing device 130 via the network 120. More descriptions regarding the collision warning may be found in FIG. 7 and the related descriptions.

The network 120 may include any suitable network that facilitates information and/or data exchange of the intelligent control system 100. In some embodiments, one or more components (e.g., the medical device 110, the processing device 130, the storage device 140, or the voice device 150) of the intelligent control system 100 transmit the information and/or data to one or more other components of the intelligent control system 100 via the network 120. For example, the processing device 130 obtains a medical image of the target object (e.g., the patient) from the medical device 110 via the network 120. In some embodiments, the network 120 is any one or more of a wired network or a wireless network. In some embodiments, the network has various topologies such as point-to-point, shared, centralized, or the like, or a combination of a plurality of topologies.

The processing device 130 may process the data and/or information obtained from the medical device 110, the storage device 140, and/or the voice device 150. In some embodiments, the processing device 130 is configured to process a voice signal to obtain a control instruction for controlling the target device. For example, the processing device 130 obtains a first voice signal. As another example, the processing device 130 performs a voice recognition on the first voice signal to obtain a voice recognition result. As another example, the processing device 130 determines, based on the voice recognition result, whether the first user voice includes the control instruction for the target device in the examination room. As another example, in response to determining that the first user voice includes the control instruction for the target device in the examination room, the processing device 130 controls the target device based on the control instruction. In some embodiments, the target device includes the display device 170 and/or the medical device 110. The display device 170 may be configured to determine a display content and a display manner of the display device 170 based on a part of the control instruction. The medical device 110 may be configured to control an operation of the medical device 110 based on at least another part of the control instruction.

In some embodiments, the processing device 130 is a single server or a server group. The server group may be centralized or distributed. In some embodiments, the processing device 130 is local or remote. The processing device 130 may be directly connected to the medical device 110, the storage device 140, and the voice device 150 to access stored or obtained information and/or data. In some embodiments, the processing device 130 is implemented on a cloud platform. Merely by way of example, the cloud platform includes a private cloud, a public cloud, a hybrid cloud, a community cloud, a distributed cloud, an internal cloud, a multi-layer cloud, or the like, or any combination thereof.

The storage device 140 stores data and/or instructions. In some embodiments, the storage device 140 stores data obtained from the medical device 110, the terminal 130, and/or the processing device 130. For example, the storage device 140 stores the medical image obtained by the medical device 110, etc. In some embodiments, the storage device 140 stores the data and/or instructions that the processing device 130 executes or uses to perform the exemplary methods described in the present disclosure. For example, the storage device 140 stores the instructions for the processing device 130 to perform the methods shown in the flowcharts. In some embodiments, the storage device 140 includes a mass storage device, a removable storage device, a volatile read-write memory, a read-only memory (ROM), or the like, or any combination thereof. In some embodiments, the storage device 140 is implemented on the cloud platform.

In some embodiments, the storage device 140 is connected to the network 120 to communicate with one or more components (e.g., the medical device 110, the processing device 130, the voice device 150, etc.) of the intelligent control system 100. One or more components of the intelligent control system 100 accesses the data or instructions stored in the storage device 140 via the network 120. In some embodiments, the storage device 140 is directly connected to or directly communicates with one or more components of the intelligent control system 100. In some embodiments, the storage device 140 may be part of the processing device 130.

In some embodiments, the processing device 130 and/or the storage device 140 may be part of the medical device 110.

The voice device 150 is used to implement a voice interaction between a user and the intelligent control system 100. For example, the voice device 150 is configured to detect a user voice and convert the detected user voice into the voice signal. In some embodiments, the voice device 150 includes a voice input component (e.g., a microphone) and a voice output component (e.g., a speaker). The microphone is configured to receive the user voice (e.g., the first user voice, a second user voice), and the speaker is configured to play a feedback voice. An exemplary microphone includes a dynamic microphone, a capacitor microphone, an electret microphone, a silicon microphone, a liquid microphone, a laser microphone, or the like, or any combination thereof. An exemplary speaker includes a dynamic (electrodynamic) speaker, a capacitor (electrostatic) speaker, a piezoelectric (crystal or ceramic) speaker, an electromagnetic (magnetic armature) speaker, an electro-ion speaker, a pneumatic speaker, or the like, or any combination thereof.

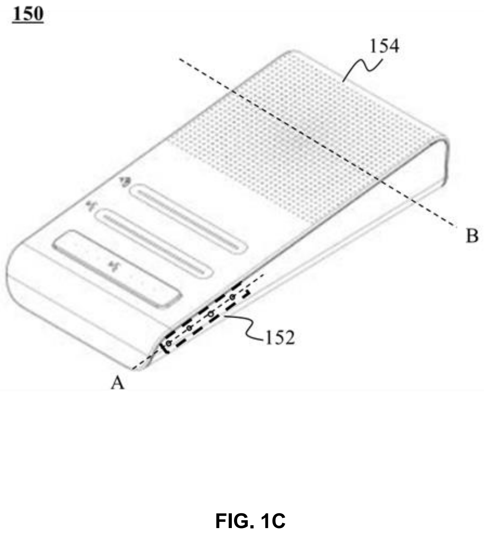

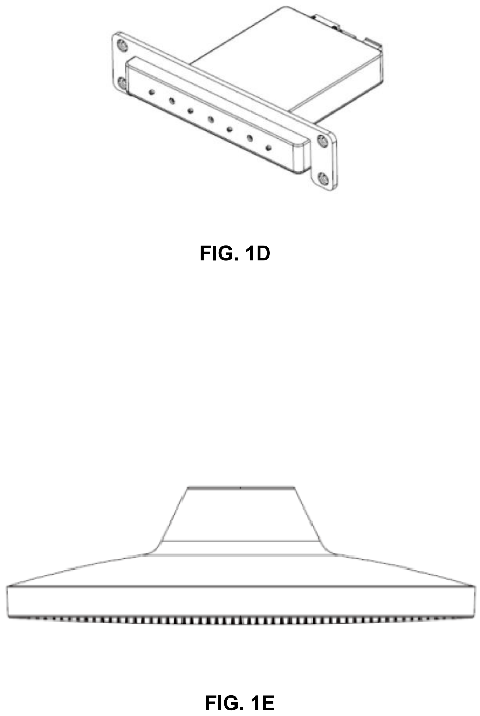

In some embodiments, the microphone and the speaker are integrated on the same voice device 150. For example, as shown in FIG. 1C, a microphone 152 and a speaker 154 may be integrally arranged on the voice device 150. That is, the microphone 152 and the speaker 154 are integral structures. In some embodiments, the microphone and the speaker are independent devices. For example, the voice device 150 includes one or more microphone devices as shown in FIG. 1D and one or more speaker devices as shown in FIG. 1E.

In some embodiments, a count and a type of the microphone and/or the speaker are determined according to actual situations. For example, in the examination room, four microphones and one speaker are arranged separately. In a control room, four microphones and one speaker are arranged integrally. As another example, when an ambient noise is greater than a noise threshold (e.g., 100 decibels (dB)), and a distance between the user and the microphone is less than a first distance threshold (e.g., 10 centimeters, 20 centimeters, 30 centimeters, 50 centimeters, etc.), a gooseneck microphone is used. As yet another example, when the ambient noise is greater than the noise threshold, and the distance between the user and the microphone is greater than a second distance threshold (e.g., 1 meter, 2 meters, 3 meters, 5 meters, etc.), a plurality of microphones (e.g., 128 microphones) are used for sound directional enhancement, thereby enhancing a sound in a specific region and weakening surrounding the noise.

In some embodiments, a plurality of microphones (or speakers) may be arranged in a certain array manner. For example, four microphones are arranged equidistantly as shown in FIG. 1C. As another example, a plurality of microphones (or speakers) are arranged in a regular shape (e.g., a circle, a rectangle, a square, a triangle, an ellipse, etc.) or an irregular shape. In some embodiments, the arrangements of the microphones and the speakers need to satisfy certain conditions. For example, a minimum spacing between the microphone and the speaker needs to be greater than a third distance threshold (e.g., 5 centimeters, 10 centimeters, 15 centimeters, etc.). As another example, a central axis of a plurality of microphones and a central axis of a plurality of speakers need to be placed vertically or approximately vertically. Merely by way of example, as shown in FIG. 1C, a projection of the voice device 150 is approximately a rectangle, a central axis of the microphone 152 (as shown by dashed line A) is parallel to a long side of the rectangle, and a central axis of the speaker 154 (as shown by dashed line B) is parallel to a short side of the rectangle.

In some embodiments, the voice device 150 is a part of the processing device 130. For example, the voice device 150 is integrated in the processing device 130 as an operation console of the medical device 110.

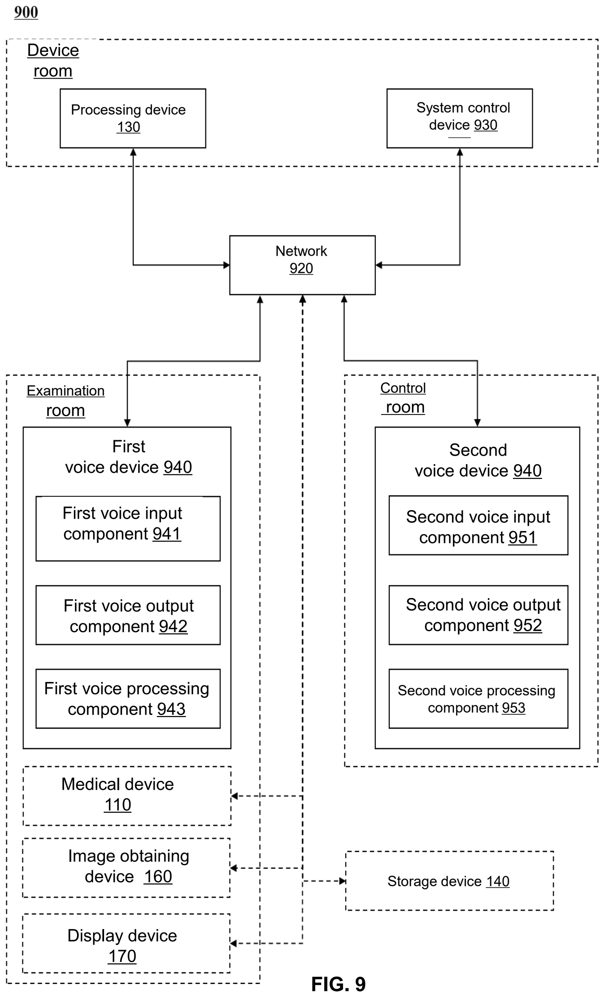

In some embodiments, the voice device 150 includes a plurality of sub-devices. For example, the voice device includes a first voice device and/or a second voice device. The first voice device may be arranged in the examination room, and on one or more of the display device 170 and the medical device 110. The second voice device may be arranged in the control room. The second voice device may be communicatively connected to the first voice device. More descriptions regarding the first voice device and the second voice device may be found in FIGs. 9-14 and the descriptions thereof.

The image obtaining device 160 may be used to obtain a real-time optical image of the examination room (e.g., a first real-time optical image, a second real-time optical image, a third real-time optical image). The image obtaining device 160 may include a camera. In some embodiments, the image obtaining device 160 includes a plurality of devices installed at different positions to obtain the real-time optical images of the same region from different shooting angles and/or the real-time optical images of different regions. The image obtaining device 160 includes a camera (e.g., a digital camera, an analog camera, a depth camera, a structured light camera, etc.), a sensor (e.g., a red-green-blue (RGB) sensor, an RGB-depth (RGB-D) sensor, etc.), a laser imaging device (e.g., an phase laser collection device, a point laser acquisition device, etc.), and various other devices capable of collecting optical image data of the target object.

The display device 170 may be used to display relevant information of various medical links in the medical procedure. For example, the display device 170 displays the real-time optical image(s). As another example, the display device 170 displays the medical image, change information of physiological indicators (e.g., blood pressure, heart rate, etc.) or a graph, etc. of the patient in the medical procedure. As yet another example, the display device 170 displays feedback information (e.g., content of the feedback voice) after the control instruction is executed. The display device 170 may include a liquid crystal display (LCD), a light-emitting diode (LED)-based display, a flat panel display, a curved display, a television device, a cathode ray tube (CRT), or the like, or any combination thereof. In some embodiments, the display device 170 and the medical device 110 are arranged adjacent to each other.

In some embodiments, the voice device 150, the image obtaining device 160, and/or the display device 170 are integrated on a same device. In some embodiments, the voice device 150, the image obtaining device 160, and the display device 170 are different devices.

In some embodiments, the voice device 150, the image obtaining device 160, and/or the display device 170 are installed at any position in the examination room. For example, the voice device 150, the image obtaining device 160, and/or the display device 170 are fixed at a certain fixed position (e.g., a ceiling) in the examination room through a detachable or non-detachable connection manner. As another example, the examination room is configured with a slide rail, and the voice device 150, the image obtaining device 160, and/or the display device 170 are installed on the slide rail through the detachable or non-detachable connection manner, so that positions of the voice device 150, the image obtaining device 160, and/or the display device 170 can be flexibly adjusted according to a position of the user.

In some embodiments, the intelligent control system 100 further includes a system control device (not shown in the figure). The system control device refers to a device configured to control the target device (e.g., the medical device 110, the voice device 150, the image obtaining device 160, and/or the display device 170). In some embodiments, the system control device includes a system controller unit (SCU) configured to execute the control instruction related to the target device. For example, through the system controller unit, the system control device controls a gantry of the medical device 110 to move to a specific position, and adjusts one or more parameters (e.g., a ray switch, etc.) of the medical device 110.

In some embodiments, the system control device includes a plurality of system controller units set for different medical procedures. For example, the system control device includes a system controller unit for X-ray imaging, a system controller unit for DSA examination, etc. Different system controller units may include different control instruction sets, which are predetermined according to the medical procedure (e.g., preset according to a scan protocol). The system controller unit of the system control device executes one or more control instructions to implement an operation of the target device. In some embodiments, the system control device is a part of the medical device 110. In some embodiments, the system control device and the processing device 130 are integrated into one device.

In some embodiments, components of the intelligent control system 100 are disposed in a same room. For example, as shown in FIG. 1A, the medical device 110, the network 120, the processing device 130, the storage device 140, the voice device 150, the image obtaining device 160, and the display device 170 are arranged in the examination room. In some embodiments, some components of the intelligent control system 100 are arranged in different rooms. More descriptions regarding the components of the intelligent control system arranged in different rooms may be found in FIGs. 9-14 and the descriptions thereof.

It should be noted that the intelligent control system 100 is provided merely for illustration purposes and is not intended to limit the scope of the present disclosure. For those of ordinary skilled in the art, various modifications or changes may be made according to the description of the present disclosure. For example, the intelligent control system 100 further includes a database, an information source, etc. As another example, the intelligent control system 100 is implemented on other devices to achieve similar or different functions. However, these changes and modifications do not depart from the scope of the present disclosure.

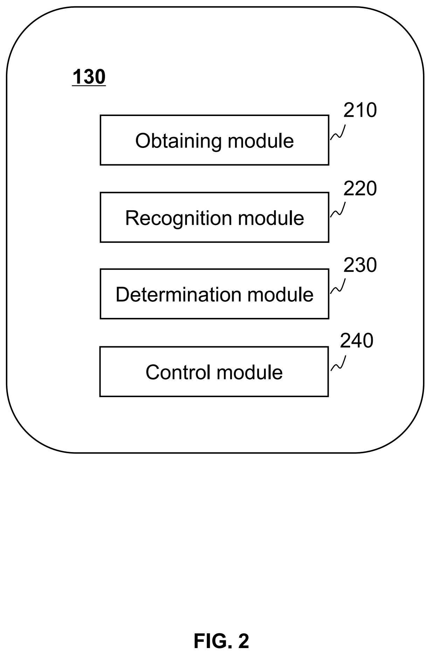

FIG. 2 is a block diagram illustrating an exemplary processing device according to some embodiments of the present disclosure. The processing device 130 may include an obtaining module 210, a recognition module 220, a determination module 230, and a control module 240.

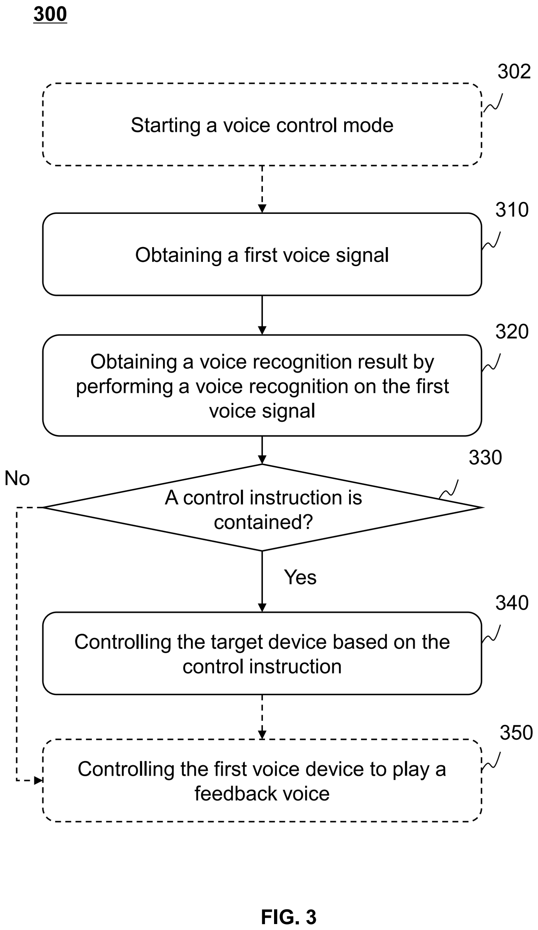

The obtaining module 210 may be configured to obtain a first voice signal. The first voice signal may be generated by a first voice device by detecting a first user voice, and the first voice device may be installed in an examination room. More descriptions regarding obtaining the first voice signal may be found in operation 310 of FIG. 3 and related descriptions thereof.

The recognition module 220 may be configured to perform a voice recognition on the first voice signal to obtain a voice recognition result. More descriptions regarding obtaining the voice recognition result may be found in operation 320 of FIG. 3 and related descriptions thereof.

The determination module 230 may be configured to determine, based on the voice recognition result, whether the first user voice includes a control instruction for a target device in the examination room. The target device may include a display device (e.g., the display device 170) and/or a medical device (e.g., the medical device 110) installed in the examination room. More descriptions regarding determining whether the first user voice includes the control instruction for the target device in the examination room may be found in operation 330 of FIG. 3 and related descriptions thereof.

The control module 240 may be configured to, in response to determining that the first user voice includes the control instruction for the target device in the examination room, control the target device based on the control instruction. More descriptions regarding controlling the target device may be found in operation 340 of FIG. 3 and related descriptions thereof.

Each module in the processing device 130 may be implemented in whole or in part by software, hardware, and a combination thereof. Each module may be embedded in a processor of a computer device in a hardware form or be independent from the processor of the computer device, or may be stored in a memory of the computer device in a software form, so that the processor may invoke and execute operations corresponding to each module.

It should be noted that the above descriptions of the processing device 130 and modules thereof are for convenience of description only and do not limit the present disclosure to the embodiments described. It may be understood that for those skilled in the art, after understanding the principle of the processing device 130, various modules may be combined arbitrarily or constitute sub-devices to connect with other modules without departing from the principle. For example, the obtaining module 210, the recognition module 220, the determination module 230, and the control module 240 disclosed in FIG. 2 are different modules in one system, or one module implements functions of the above two or more modules. As another example, each module in the processing device 130 shares one storage module, or each module has its own storage module. Such modifications are within the protection scope of the present disclosure.

FIG. 3 is a flowchart illustrating an exemplary intelligent control process according to some embodiments of the present disclosure. In some embodiments, process 300 is performed by the intelligent control system 100. For example, process 300 is stored in a storage device (e.g., the storage device 140) in a form of an instruction set (e.g., an application). In some embodiments, the processing device 130 (e.g., one or more modules shown in FIG. 2) executes the instruction set and accordingly instructs one or more components of the intelligent control system 100 to perform process 300.

A medical procedure typically requires collaboration among a plurality of individuals. For example, a doctor in an examination room (e.g., a scan room or an operation room) needs to manually operate a control box and/or a touch panel of a medical device to control the medical device while performing an examination or surgery, which may easily interrupt the examination or surgery and even affect a patient safety in emergency situations. As another example, the doctor needs to communicate with a technician to have the technician assist with operations related to the medical procedure, which imposes high requirements on the technician's professional skills, proficiency, and coordination with the doctor. Furthermore, each operation in the medical procedure requires a plurality of rounds of feedback between the doctor and the technician, which significantly impacts the execution efficiency and safety of the medical procedure.

Therefore, there is a need to provide a method for intelligent control to assist a user in controlling a target device via voice, thereby improving the execution efficiency and safety of the medical procedure. In some embodiments, the intelligent control is implemented by performing the following operations of process 300.

In operation 310, the processing device 130 (e.g., the obtaining module 210) obtains a first voice signal.

The first voice signal may be generated by a first voice device by detecting the first user voice. The first voice device may be installed in the examination room. More descriptions regarding the voice device may be found in FIGs. 1A, 1C-1E, and 9-14 and their descriptions.

The first user voice refers to a voice emitted by at least one user (also referred to as a first user) in the examination room. The at least one user may include the doctor (e.g., a surgeon), a nurse, a technician (e.g., a technician assisting the surgeon, a call-out technician), etc., performing the medical procedure, or any combination thereof. In some embodiments, the first user voice is emitted before or during the execution of the medical procedure.

The medical procedure may be a scanning procedure, a surgical procedure, a treatment procedure, etc., which includes a plurality of medical operations. Merely by way of example, the medical procedure includes a preparation operation for a scan plan, a scan execution operation, a preparation operation for treatment or surgery, an execution operation for treatment or surgery, a post-operative treatment operation, or the like, or any combination thereof. There are different types of medical procedures, which are determined based on different medical scenarios (e.g., a region of interest for scan, a type of surgery). For example, a DSA surgery scenario corresponds to a DSA medical procedure. In some embodiments, the medical operations included in different types of medical procedures are predetermined.

The first voice signal may be a digital signal. For example, the first voice device detects or collects the first user voice and converts the first user voice into the first voice signal for subsequent processing. As another example, the first voice device detects or collects the first user voice, converts the first user voice into an initial voice signal, and then processes the initial voice signal to generate a voice signal. The processing at least includes a noise reduction processing.

It can be understood that various types of noise may exist in the voice detection environment (e.g., the examination room), such as various device noises (e.g., mechanical noise), environmental noises (e.g., traffic noise), etc. The initial voice signal refers to an initial digital signal generated by converting the first user voice. The noise reduction processing refers to a process of removing a noise signal from the initial voice signal. The noise reduction processing may be performed based on various audio noise reduction algorithms, such as a time-domain filtering algorithm, a frequency-domain filtering algorithm, etc. For example, the first voice device performs the noise reduction processing on the initial voice signal. In some embodiments, the first voice device first performs the noise reduction processing on received first user voice and then converts the noise-reduced voice into the first voice signal.

Compared with performing the noise reduction processing by the processing device 130, performing the noise reduction processing on the first user voice by the first voice device can reduce a computational load on the processing device 130.

In some embodiments, the processing device 130 obtains the first voice signal from the first voice device (e.g., the voice device 150). Alternatively, the processing device 130 may obtain the first voice signal from the storage device (e.g., the storage device 140, an external storage device) storing the first voice signal.

In operation 320, the processing device 130 (e.g., the recognition module 220) obtains a voice recognition result by performing a voice recognition on the first voice signal.

The voice recognition result may be in a form of text, which indicates a voice content of the first user voice. In some embodiments, the processing device 130 processes the first voice signal using various voice recognition technologies to obtain the voice recognition result. For example, the processing device 130 processes the first voice signal using, for example, a language processing model, to obtain the voice recognition result. The language processing model includes one or a combination of acoustic models such as a Hidden Markov Model (HMM), a Bidirectional Encoder Representations from Transformer (BERT) model, a Generative Pre-Trained Transformer (GPT) model, etc. In some embodiments, the language processing model is a large language model.

In some embodiments, the language processing model is trained separately for different medical procedures or different medical scenarios. For example, the language processing model includes a model for a DSA surgery scenario, a model for a lung tumor resection surgery scenario, etc., and also includes a model for a specific doctor (e.g., the surgeon) or for a technician (e.g., a call-out technician) in a specific medical scenario (e.g., a DSA surgery scenario). It may be understood that training different language processing models for different medical procedures and different users can improve the accuracy of voice recognition.

In some embodiments, the voice recognition result may be in other forms recognizable by the processing device 130, for example, an electrical signal, a code, an encoded instruction, etc., indicating a voice content of the first user voice.

In some embodiments, the voice recognition result includes a control instruction related to the target device. Merely by way of example, the voice recognition result is "move the gantry to a left position," etc. The voice recognition result may also include other communication contents, such as instructions from the doctor to the patient, daily communication among medical workers, etc.

In some embodiments, the voice recognition result indicates a speech content of a target user. The target user refers to a preset user who is permitted to control the medical procedure. For example, the target user is a user permitted to adjust parameters of a scan device, a user permitted to control a start or stop of a medical scan, etc. In some embodiments, the target user is determined based on a medical protocol (e.g., a scan protocol, an examination protocol) related to the medical procedure.

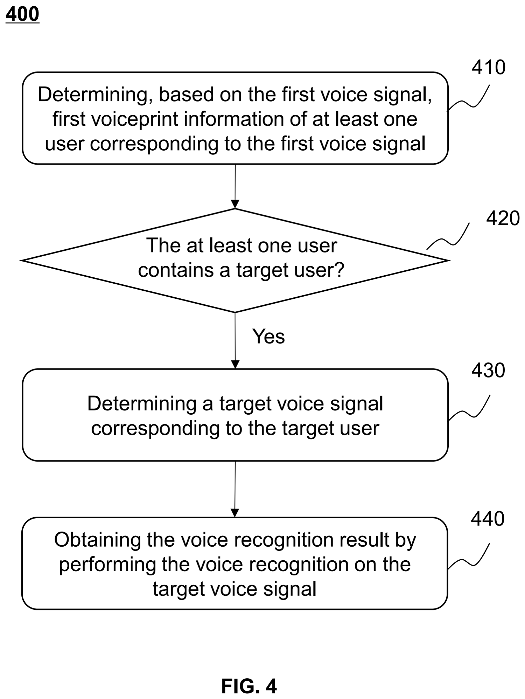

In some embodiments, the processing device 130 determines first voiceprint information of at least one user corresponding to the first voice signal based on the first voice signal. The processing device 130 determines whether the at least one user contains the target user based on the first voiceprint information of the at least one user. In response to determining that the at least one user includes the target user, the processing device 130 determines a target voice signal corresponding to the target user, and performs a voice recognition on the target voice signal to obtain the voice recognition result. More descriptions regarding performing the voice recognition on the target voice signal to obtain the voice recognition result may be found in FIG. 4 and the descriptions thereof.

In operation 330, the processing device 130 (e.g., the determination module 230) determines whether the first user voice contains a control instruction for a target device in an examination room based on the voice recognition result.

The target device refers to a device that a user (e.g., the target user) wants to control. For example, the target device includes one or more of the medical device 110, the voice device 150, the image obtaining device 160, and the display device 170 in FIG. 1A.

The control instruction refers to an instruction for controlling the target device to perform a specific operation. For example, when the target device includes the display device installed in the examination room, the control instruction includes an instruction for switching a display image on the display device, an instruction for playing or pausing playing the display image on the display device, an instruction for switching a display mode (e.g., a roadmap mode) of the display device, an instruction for setting a display parameter (e.g., an image size, a resolution, a presentation angle) of the display image on the display device, an instruction for processing the display image on the display device, an instruction for setting a segmentation manner of a display region of the display device, an instruction for making a display interface to jump to a user configuration interface, or the like, or any combination thereof. Merely by way of example, the processing performed on the display image includes saving the display image, sending the display image to a user terminal, performing the noise reduction processing on the display image, performing a subtraction operation on the display image, etc.

As another example, when the target device includes the medical device (e.g., an X-ray imaging device) installed in the examination room, the control instruction includes an instruction for performing a motion control on a component (e.g., the gantry, a patient bed) of the medical device, an instruction for making the medical device to perform the medical procedure on a target region of the target object, an instruction for switching or setting an operating mode (e.g., a DSA mode, a GCT mode) of the medical device, an instruction for setting a relevant parameter (e.g., a scan parameter, a radiotherapy parameter) of the medical procedure corresponding to the medical device, an instruction for making the medical device to start or stop the medical procedure, or the like, or any combination thereof.

As another example, when the target device includes an image obtaining device (e.g., the image obtaining device 160) installed in the examination room, the control instruction includes an instruction for performing a motion control on the image obtaining device, an instruction for adjusting a shooting parameter (e.g., a focal length, an exposure time) of the image obtaining device, an instruction for making the image obtaining device to send a captured real-time optical image, or the like, or any combination thereof.

As another example, when the target device includes a voice device (e.g., the voice device 150) installed in the examination room, the control instruction includes an instruction for performing a motion control on the voice device, an instruction for adjusting a collection parameter (e.g., a position, an orientation, noise reduction of a microphone) of the voice device, an instruction for adjusting a playback parameter (e.g., a position, an orientation, a volume of a speaker) of the voice device, or the like, or any combination thereof.

In some embodiments, the processing device 130 performs preprocessing on the voice recognition result. For example, the processing device 130 processes the voice recognition result based on an information extraction technology to determine a keyword included in the voice recognition result, and further determines whether the voice recognition result includes the control instruction related to the target device. The keyword may include the target device and one or more components thereof. As another example, the processing device 130 compares the voice recognition result with a preset keyword corresponding to the target device. If the voice recognition result includes the preset keyword, it may be determined that the first user voice includes the control instruction for the target device.

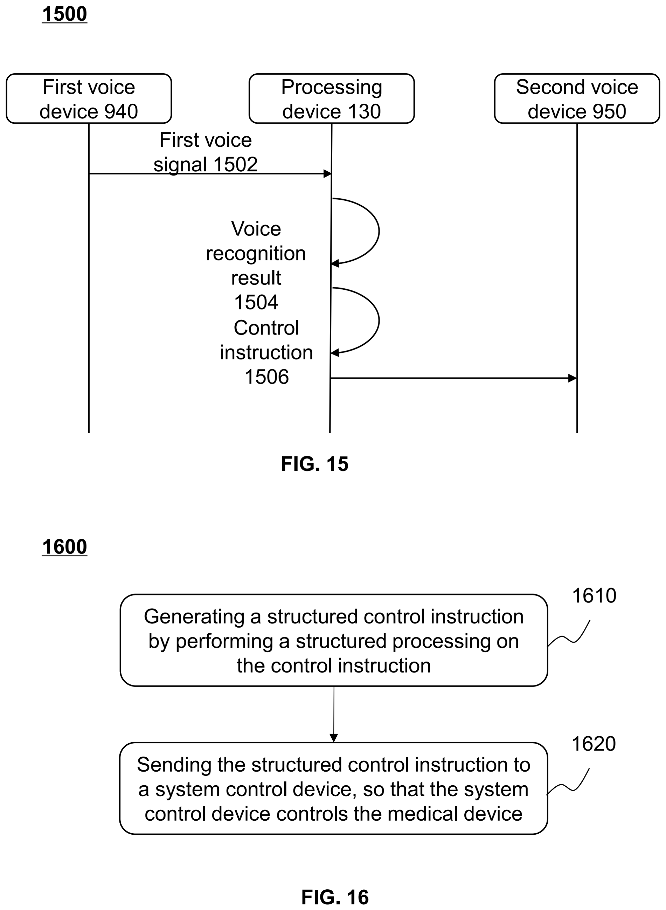

In some embodiments, the processing device 130 further performs a structured processing on the voice recognition result to determine a structured control instruction, and then determines whether the voice recognition result includes the control instruction based on a preset control instruction set and the structured control instruction corresponding to the voice recognition result. More descriptions regarding determining whether the voice recognition result includes the control instruction may be found in FIG. 16 and the descriptions thereof.

If it is determined that the first user voice includes the control instruction for the target device in the examination room, the processing device 130 may perform operation 340. If it is determined that the first user voice does not include the control instruction for the target device in the examination room, the processing device 130 may perform operation 350 or terminate process 300.

In operation 340, the processing device 130 (e.g., the control module 240) controls the target device based on the control instruction.

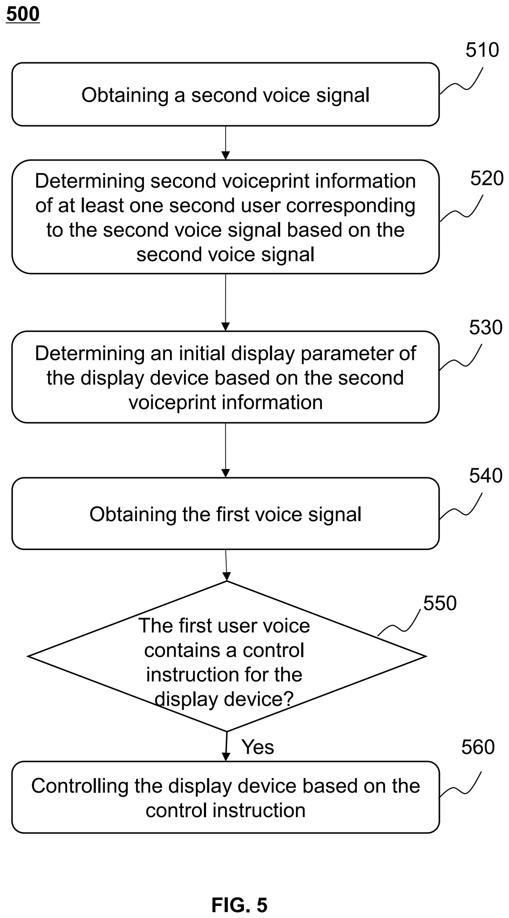

For example, if the control instruction is the control instruction for the display device 170, the processing device 130 controls the display device 170 based on the control instruction. Correspondingly, in response to the control instruction, the display device 170 may perform at least one of the following operations: switching the display image on the display device 170, playing or pausing playing the display image on the display device 170, switching the display mode of the display device 170, setting the display parameter of the display image on the display device 170, processing the display image on the display device 170, setting the segmentation manner of the display region of the display device 170, making the display interface to jump to the user configuration interface, etc. More descriptions regarding controlling the display device 170 based on the control instruction may be found in FIGs. 5-6B and the descriptions thereof.

As another example, if the control instruction is the control instruction for the medical device 110, the processing device 130 controls the medical device 110 based on the control instruction. Correspondingly, in response to the control instruction, the medical device 110 may perform at least one of the following operations: controlling the component of the medical device 110 to move, performing the medical procedure on the target region of the target object, setting an operating mode of the medical device 110, setting a relevant parameter of the medical procedure corresponding to the medical device 110, starting or stopping the medical procedure, etc. More descriptions regarding controlling the medical device 110 based on the control instruction may be found in FIGs. 7-8B and the descriptions thereof.

In some embodiments, based on the control instruction, the processing device 130 further controls the display device 170 to display the operation performed by the medical device 110. For example, in response to the control instruction, the display device 170 displays the medical procedure performed by the medical device 110.

In some embodiments, the processing device 130 determines whether the first user voice includes a wake-up word based on the voice recognition result. The wake-up word may indicate that the first user voice is related to a voice control mode. The wake-up word may be a preset word used to distinguish daily communication contents, for example, A01, B02, etc. The description of the wake-up word herein is merely for example and is not intended to limit the form of the wake-up word. If it is determined that the first user voice includes the wake-up word and includes the control instruction for the target device, the processing device 130 may control the target device based on the control instruction. That is, the user needs to utter both the wake-up word and the control instruction to control the target device. In this way, a security of control can be ensured.

In some embodiments, after the voice control mode is turned on, or within a preset time period after the user utters the wake-up word, the user does not need to utter the wake-up word and only needs to utter the control instruction to control the target device. Thereby, repeated wake-up operations may be avoided, and a control efficiency can be improved.

In some embodiments, the processing device 130 sends the control instruction to a system control device, so that the system control device controls the target device (e.g., the medical device). More descriptions regarding making the system control device to control the medical device may be found in FIG. 16 and the descriptions thereof.

In operation 350, the processing device 130 (e.g., the control module 240) controls the first voice device to play a feedback voice.

The feedback voice refers to a voice containing an execution situation of the control instruction. For example, the feedback voice includes a feedback voice indicating whether the control instruction has been executed (e.g., the first voice device plays "the control instruction has been executed," "the control instruction execution failed"), a feedback voice containing prompt information of a next operation (e.g., the first voice device plays "the control instruction has been executed, whether to perform imaging," "the control instruction execution failed, confirm whether the target device is abnormal"), or the like, or any combination thereof.

In some embodiments, the processing device 130 determines a security level of the control instruction based on the voice recognition result, and then determines content of the feedback voice based on the security level. Further, the processing device 130 may control the first voice device to play the feedback voice based on the content of the feedback voice. For example, types of security levels and control instructions and the feedback voice content corresponding to each security level are determined in advance, and the correspondence is stored in a form of a comparison table. The processing device 130 may determine the security level of the control instruction in the voice recognition result and the feedback voice content according to the comparison table.

Merely by way of example, the security level and the corresponding feedback voice content are determined according to a security risk of a device operation corresponding to each control instruction and a feedback situation of the device. For example, the security level is divided into four types: A, B, C, and D. Type A corresponds to a control instruction with a high security risk and no protective measures, for example, a ray unlocking, a motion unlocking, etc. The feedback voice content corresponding to the control instruction of type A may be a request for the user to perform a voice confirmation (e.g., "please confirm whether to emit rays"). Type B corresponds to a control instruction with a high security risk but with protective measures, for example, modification of parameters related to motion, radiation, protocol, etc. The feedback voice content corresponding to the control instruction of type B may include repeating the control instruction (e.g., "adjust C-arm to the correct position"), which is used to ensure that the user knows the upcoming operation. Type C corresponds to a control instruction with a low security risk but with unobvious feedback, for example, modification of parameters related to the display device, or a prompt indicating the end of radiation. The feedback voice content corresponding to the control instruction of type C may include a prompt tone corresponding to successful execution or a prompt tone corresponding to failed execution. Type D corresponds to a control instruction with a low security risk and obvious feedback, for example, an alarm cancellation. The feedback voice content corresponding to the control instruction of type D may include no additional prompt.

In some embodiments, the processing device 130 also controls the display device to display a content of the feedback voice. For example, the processing device 130 sends the feedback voice content to the display device to control the display device to display the content of the feedback voice in forms such as a text, an image, a color, etc.

In some embodiments, the feedback voice also includes a response to an inquiry in the first user voice. For example, the processing device 130 determines, based on the voice recognition result, whether the first user voice includes the inquiry. In response to determining that the first user voice includes the inquiry, the processing device 130 may determine response information corresponding to the inquiry, and control the first voice device to play the response information. In some embodiments, the processing device 130 also controls the display device to display the response information.

In some embodiments, the feedback voice also includes a prompt for an abnormal situation. For example, the processing device 130 obtains abnormal feature information of the target object, and control the first voice device to play the abnormal feature information. In some embodiments, the processing device 130 also controls the display device to display the abnormal feature information.

In some embodiments of the present disclosure, by determining whether the first user voice includes the control instruction for the target device, an intelligent voice control of the target device is achieved. Therefore, the doctor can directly perform a voice interaction with the target device without manually controlling the target device, thereby reducing a number of medical workers required in the medical procedure, improving the execution efficiency and accuracy of the medical procedure, and ensuring that the medical procedure proceeds more smoothly.

It should be noted that the above description of process 300 is provided for illustrative purposes only and is not intended to limit the scope of the present disclosure. For those of ordinary skill in the art, various changes and modifications can be made under the guidance of the present disclosure. However, these changes and modifications do not depart from the scope of the present disclosure. In some embodiments, process 300 is completed through one or more additional operations not described and/or omitting one or more of the operations discussed above.

For example, before operation 310, process 300 includes operation 302, where the processing device 130 starts a voice control mode. Specifically, the processing device 130 may obtain a second voice signal, and determine whether the second user voice includes the wake-up word. In response to determining that the second user voice includes the wake-up word, the processing device 130 starts the voice control mode. The second voice signal may be generated by the first voice device by detecting the second user voice.

FIG. 4 is a flowchart illustrating an exemplary voice recognition process according to some embodiments of the present disclosure. In some embodiments, operation 320 in FIG. 3 is implemented by performing process 400.

In operation 410, the processing device 130 (e.g., the recognition module 220) determines, based on the first voice signal, first voiceprint information of at least one user corresponding to the first voice signal.

The first voiceprint information refers to voice feature information of the at least one user who uttered the first user voice, which is used to determine an identity of the at least one user, for example, a specific doctor, technician, nurse, etc.

In some embodiments, the processing device 130 performs a voiceprint feature extraction processing on the first voice signal based on a voiceprint recognition technology to obtain the first voiceprint information of the at least one user corresponding to the first voice signal.

In operation 420, the processing device 130 (e.g., the recognition module 220) determines, based on the first voiceprint information of the at least one user, whether the at least one user contains a target user.

In some embodiments, the processing device 130 obtains target voiceprint information corresponding to the target user. The target voiceprint information refers to voiceprint information (e.g., a voiceprint feature vector) of the target user. For example, the processing device 130 obtains the target voiceprint information corresponding to the target user from a voiceprint feature library. The voiceprint feature library may store voiceprint information of a plurality of users (e.g., doctors, nurses, technicians, etc. in a hospital). For example, voices of different doctors, medical workers, etc. are collected in advance, and the voiceprint information corresponding to each user is generated based on the voiceprint extraction technology, thereby generating the voiceprint feature library based on identity information (e.g., name, employee number, etc.) of each user and the corresponding voiceprint information. The voiceprint feature library may be stored on the storage device 140, or may be stored on an independent storage device.

In some embodiments, the processing device 130 determines whether the at least one user includes the target user by comparing the first voiceprint information of the at least one user with the target voiceprint information. For example, the processing device 130 performs a voiceprint matching processing on the first voiceprint information of each of the at least one user and the target voiceprint information to determine whether the user is the target user. For example, the processing device 130 respectively determines a similarity between the first voiceprint information of each user and the target voiceprint information, and determines whether the similarity is greater than a preset similarity threshold. In response to determining that the similarity between the first voiceprint information of the user and the target voiceprint information of a certain target user is greater than the preset similarity threshold, the user is determined to be the target user.

In some embodiments, for each of the at least one user, the processing device 130 determines the identity information of the user based on the first voiceprint information, and determines a discourse weight of the user based on the identity information of the user. Further, the processing device 130 determines whether the user is the target user based on the discourse weight of the user. For example, the processing device 130 determines the discourse weight of each user in advance based on user work information (e.g., a position, a working year, a number of surgeries performed, etc.), and stores the discourse weight in the storage device (e.g., the storage device 140, the independent storage device). After the identity information of each user in the at least one user is determined based on the first voiceprint information, the discourse weight of each user may be determined, and whether the discourse weight is greater than a preset weight threshold may be determined. In response to determining that the discourse weight of the user is greater than the preset weight threshold, the user is determined to be the target user.

In response to determining that the at least one user includes the target user, the processing device 130 performs operation 430. In response to determining that the at least one user does not include the target user, the processing device 130 may terminate process 400. In some embodiments, in response to determining that the at least one user does not include the target user, the processing device 130 determines that the first user voice does not include a control instruction for the target device in an examination room.

In operation 430, the processing device 130 (e.g., the recognition module 220) determines a target voice signal corresponding to the target user.

In some embodiments, the processing device 130 determines a voice signal corresponding to the target user as the target voice signal. For example, if the at least one user only includes the target user, the processing device 130 determines the first voice signal as the target voice signal. As another example, if the at least one user includes the target user and other users, the processing device 130 uses a voice signal extraction algorithm to extract the voice signal corresponding to the target user from the first voice signal, and determines the voice signal corresponding to the target user as the target voice signal.

In operation 440, the processing device 130 (e.g., the recognition module 220) obtains the voice recognition result by performing the voice recognition on the target voice signal.

In some embodiments, the processing device 130 may process the target voice signal through various voice recognition technologies to obtain the voice recognition result. More descriptions regarding the voice recognition technologies may be found in operation 320.

In some embodiments of the present disclosure, the identity of a speaking user is identified through voiceprint information analysis, and only the target voice signal corresponding to the target user is extracted for voice recognition. Accordingly, an impact of non-target user voices on medical procedure control is avoided, the accuracy and security of medical device operation control can be improved, and thus the medical procedure can proceed more smoothly.

FIG. 5 is a flowchart illustrating an exemplary intelligent control process for a display device according to some embodiments of the present disclosure. In some embodiments, process 500 is a specific embodiment of process 300.

A medical procedure often requires a use of a display device to show display contents corresponding to various medical links (e.g., pre-execution, during execution, etc.) to assist doctors in medical activities (e.g., medical scans, surgeries). However, a display configuration, a display content, etc. of a display interface are often complex, and adjustments are often frequent, and different users (e.g., doctors) have vastly different usage habits and configuration preferences. Manual adjustments are often time-consuming and laborious. Especially for a medical worker unfamiliar with functions or functional interfaces of the display interface, a significant amount of time is required for system usage training. During tense medical activities, adjustments are also prone to errors, which seriously affects a normal progress of the medical activities. Therefore, there is a need to provide effective systems and methods for intelligently controlling the display device. In some embodiments, the display device is intelligently controlled by performing the following operations of process 500.

In operation 510, the processing device 130 (e.g., the obtaining module 210) obtains a second voice signal.

The second voice signal is generated by a first voice device detecting a second user voice. A manner of obtaining the second voice signal may be similar to a manner of obtaining the first voice signal, which is not repeated here.

The second user voice refers to a voice emitted by at least one second user participating in the medical procedure. For example, the second user voice may be a voice emitted by a person such as a doctor, a nurse, or a technician participating in the medical procedure.

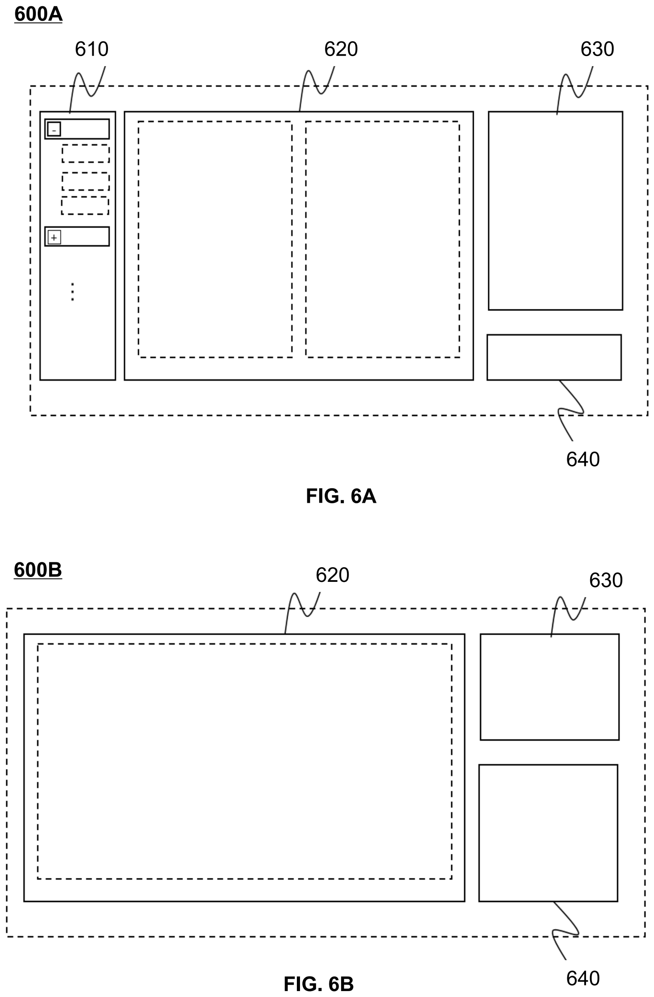

In some embodiments, the second user voice is emitted before an execution of the medical procedure. The second voice signal may be used to perform an initial configuration of the display interface of the display device (e.g., the display device 170) before the execution of the medical procedure. The initial configuration may include determining initialization parameters of the display interface and generating an initial display interface. Related content regarding the initial display interface may be found in FIG. 6A and the descriptions thereof.

In operation 520, the processing device 130 (e.g., the determination module 230) determines second voiceprint information of the at least one second user corresponding to the second voice signal based on the second voice signal.

The second voiceprint information refers to voice feature information of the at least one second user, which is used to determine an identity of the at least one second user. In some embodiments, the processing device 130 performs a voiceprint feature extraction processing on the second voice signal based on a voiceprint recognition technology etc. to obtain the second voiceprint information corresponding to the second voice signal.

In operation 530, the processing device 130 (e.g., the control module 240) determines an initial display parameter of the display device based on the second voiceprint information.

The initial display parameter refers to a parameter used to determine the initial display interface of the display device.

In some embodiments, the processing device 130 presents different initial display interfaces according to different user display preferences. The initial display interface includes an initial segmentation scheme of a screen, which includes parameter configurations such as a number, a size, and an arrangement of initial display regions in the screen. The user display preference may include display parameters that the user is interested in. It is understandable that the user has different preferences for parameters such as a background, a color, a display region division, and an arrangement of the display interface. For example, users of different ages, genders, and vision levels have different preferences for sizes (e.g., length and width), positions (e.g., left side, middle, top, etc.) of interested display regions, as well as the font and size of the displayed content.

In some embodiments, the processing device 130 records and stores historical display interface configurations of different users (e.g., the display configurations for medical links before the execution of various different types of medical procedures), and determines the display preference of each user based on situations of the historical display interface configurations (e.g., a configuration record with the highest frequency of use).

In some embodiments, the processing device 130 determines an identity of the at least one second user based on the second voiceprint information, and determines a display preference of the at least one second user according to the identity, thereby determining the initial display parameters related to the display interface to complete the initialization of the display interface. In some embodiments, the processing device 130 may determine the identity of the at least one second user based on the second voiceprint information, and determine a medical procedure to be executed according to the identity of the at least one second user. Further, the processing device 130 may determine the initial display parameters related to the display interface based on the medical procedure to be executed, to complete the initialization of the display interface.

In some embodiments, the processing device 130 determines, based on the second voiceprint information, whether the at least one second user includes a target user. The target user refers to a user among the at least one second user participating in the medical procedure who has an operation authority for the display device. In some embodiments, different operation authorities are configured for different target users. For the same operation authority, different discourse weights are configured for different target users, and instruction from the target user with a higher discourse weight needs to be processed with priority. In some embodiments, a manner of determining whether the at least one second user includes the target user is similar to the manner of determining whether the at least one first user includes the target user described in operation 420.

If it is determined that the at least one second user includes the target user, the processing device 130 may determine the initial display parameters of the display device based on the identity information of the target user. If it is determined that the at least one second user does not include the target user, the processing device 130 may terminate process 500.

In operation 540, the processing device 130 (e.g., the obtaining module 210) obtains the first voice signal.

The first voice signal may be generated by the first voice device by detecting a first user voice. In some embodiments, the first voice signal is detected during the execution of the medical procedure (e.g., scanning, surgery). More descriptions regarding obtaining the first voice signal may be found in FIG. 3 and the descriptions thereof.

In operation 550, the processing device 130 (e.g., the determination module 230) determines whether the first user voice contains a control instruction for the display device.

The processing device 130 performs operation 320 to determine a voice recognition result, and determines whether the first user voice includes the control instruction for the display device based on the method described in operation 330.

In response to determining that the first user voice includes the control instruction for the display device, the processing device 130 may perform operation 560 to control the display device based on the control instruction.

For example, the processing device 130 determines an updated display parameter related to the display interface based on the voice recognition result, to update the display interface. The updated display parameter refers to a display parameter and a corresponding parameter value used to adjust one or more display regions of the display interface. For example, the updated display parameter is used to zoom in or out (adjust a length or a width of), translate (position coordinates of), rotate, adjust a transparency of, hide/show, etc. a specific display region. The updated display parameter may also be used to adjust or process a display content in the display region, for example, to switch, send, select, play, rotate, or zoom the display image, or to adjust a color or a font size of text in the display region. Merely by way of example, for a DSA surgical scenario (a DSA medical procedure), the updated display parameter includes saving a fluoroscopy sequence, sending a reference, selecting a specific sequence, playing/pausing, switching between adjacent frames, switching between adjacent sequences, switching subtraction original images, etc. However, the manner of updating the display parameter and the updated display parameter are not limited thereto and may be determined arbitrarily as required.

In some embodiments, for each first voice signal, the processing device 130 determines an identity of a speaker of the voice signal through voiceprint analysis, and determines whether the speaker is the target user described in operation 530. If the speaker of the first voice signal is the target user (e.g., a responsible doctor or technician for the surgery), the first voice signal is used as a target voice signal to determine the updated display parameter.

In some embodiments, the processing device 130 further determines the updated display parameter based on the discourse weight. For example, the discourse weight of a chief surgeon is set as a greater value, the discourse weight of an assisting medical worker is set as a smaller value, and the discourse weight of a user not designated in the medical procedure is set as 0. The processing device 130 may determine the updated display parameter based on the user identity corresponding to the first voice signal and the discourse weight of the user identity. For example, the first voice signal corresponding to a maximum discourse weight needs to be processed with priority; the first voice signal corresponding to a discourse weight of 0 is not responded to. Accordingly, an accuracy and an efficiency of updating the display interface can be improved, and a waste of computing resources and time caused by unnecessary updates can be avoided.

In some embodiments, the processing device 130 further generates a predicted display parameter based on an adjustment condition of the display interface by the updated display parameter. Typically, updates of different display regions in the display interface are linked. An adjustment of one display region may affect other display regions. As an example, display region A and display region B are adjacent on the left and right. If display region A is enlarged (e.g., a horizontal width is increased), display region B is reduced. The processing device 130 may determine the predicted display parameter based on a link relationship among the regions. For example, in response to an enlargement of display region A, the predicted display parameter includes narrowing display region B, lowering a transparency of display region B, or hiding display region B.

In some embodiments, the predicted display parameter is also related to a user display preference parameter. For example, the display parameter preferred by the target user is determined based on a display parameter adjustment record of the target user. In some embodiments, the predicted display parameter is related to an overall aesthetic degree of the display interface.

By using the predicted display parameter, the target user may be assisted in configuring the display interface, to reduce a workload and time of the user for an overall adjustment of the display interface, and improve user experience.

In some embodiments, the processing device 130 determines feedback information (e.g., a feedback voice, feedback display information, etc.) based on the predicted display parameter. The feedback information may be used to provide the user with update suggestions for an interface parameter. In some embodiments, the feedback information further includes other types of information. For example, the feedback information includes a device usage issue (product introduction), an operation process issue (e.g., description of a certain process of the system), a device failure (e.g., software exception information), etc. Merely by way of example, when updating the display interface, upon detecting fault information of software or hardware, the processing device 130 may remind the user via the feedback information. For example, the feedback information is played through the first voice device, and the feedback display information is displayed through the display device. In some embodiments of the present disclosure, a human-computer interaction is achieved through the feedback information, thereby providing the user with targeted suggestions or feedback, and improving the user experience.

In some embodiments, the display device includes a target display region configured to display feature information of a target object. The processing device 130 may obtain abnormal feature information of the target object, and update a display manner of the target display region based on the abnormal feature information. As an example, when updated content and/or data (e.g., key data such as a blood pressure, a blood sugar, a heart rate, etc.) in the target display region is abnormal data that is below or above a threshold value, the abnormal data in the target display region is presented in a highlighted or flashing form to attract the user's attention. In some embodiments, the processing device 130 further controls the first voice device or a wearable device worn by the target user to issue alert information. For example, the processing device 130 plays the alert information through the first voice device to notify the user, so that the user does not need to constantly watch a screen to know an occurrence of an abnormal situation in time. As another example, the processing device 130 sends the alert information to an earphone and a dedicated bracelet worn by the user, a mobile phone device of the user, etc. These devices issue the alert by voice broadcast, vibration, ringing, short message, etc. Therefore, the doctors and the medical workers can shift more attention to their own work, thereby reducing a burden on technicians and doctors and improving a work quality.

In some embodiments, the processing device 130 obtains other voice signals issued by the user during the execution of the medical procedure. For example, a third voice signal issued by the user for adjusting a component of the imaging device is received, and a position of the component is adjusted based on the third voice signal. As another example, the processing device 130 receives a voice inquiry issued by the user, determines response information corresponding to the inquiry, and controls the first voice device to play the response information.

In some embodiments, the processing device 130 identifies a control instruction related to a user configuration interface based on a fourth voice signal. The user configuration interface may display a user configuration parameter. When the control instruction related to the user configuration interface is detected, the processing device 130 may control the display device to jump to the user configuration interface, and determine the updated user configuration parameter in the user configuration interface based on the fourth voice signal, to update or adjust the user configuration parameter in the user configuration interface. An exemplary user configuration parameter may include various parameters such as a display parameter, an exposure parameter, a system parameter, a print setting parameter, etc.

In some embodiments of the present disclosure, by obtaining the fourth voice signal issued by the user during the execution of the medical procedure, the system quickly and automatically jumps to the corresponding user configuration interface and automatically completes setting of the user configuration parameter in the user configuration interface.

In some embodiments, operations 510-530 are omitted, and the processing device 130 determines the initial display parameter of the display device based on other manners. For example, the initial display parameter of the display device is a system default configuration parameter. As another example, when the first voice signal includes the control instruction corresponding to the target user, the processing device 130 determines the display parameter preferred by the target user as the initial display parameter.

FIG. 6A is a schematic diagram illustrating an exemplary display interface of a display device according to some embodiments of the present disclosure.

A display interface 600A is an initial display interface determined according to an initial display parameter. As shown in FIG. 6A, the display interface 600A may include a display region 610, a display region 620, a display region 630, and a display region 640. Different display regions may be used to present different display contents in a medical procedure. Merely by way of example, the display region 610 presents a menu, the display region 620 is used to present a real-time surgical image, the display region 630 is used to present various physiological indicators of a patient (e.g., a blood pressure, a heart rate, etc.), and the display region 640 is used to present other contents (e.g., surgical instructions or alerts, etc.).

FIG. 6B is a schematic diagram illustrating an exemplary updated display interface according to some embodiments of the present disclosure.

A display interface 600B is a display interface updated according to an updated display parameter. As shown in FIG. 6B, the display region 610 (not shown in the figure) is configured to be hidden. A lateral length of the display region 620 becomes greater, such that the region covers the original display region 610. A longitudinal height of the display region 630 is reduced. A longitudinal height of the display region 640 is increased.

In some embodiments, the user adjusts the display interface from 600A to 600B by issuing a user voice such as "hide the menu bar" or "minimize the menu bar." The intelligent control system 100 processes a voice signal corresponding to the user voice by using a trained language processing model to generate a corresponding semantic text. The semantic text may include an operation subject and an update parameter. For example, the operation subject "menu bar" corresponds to the display region 610, and the update parameter is "hide." Further, the intelligent control system 100 generates a corresponding program instruction based on the semantic text. For example, the program instruction is used to set the parameter of the display region 610 as "hidden" (e.g., a display attribute is set as false).

FIG. 7 is a flowchart illustrating an exemplary collision detection process according to some embodiments of the present disclosure.

One or more components (e.g., a C-arm gantry) of a medical device (e.g., an X-ray imaging device) usually needs to be moved relatively frequently in a medical procedure to observe a target region at different angles, so as to achieve more accurate scanning or treatment. However, as positioning takes a long time, problems such as an increase in a duration of the entire medical procedure and an increase in invalid radiation may occur. During motion of the one or more components, the one or more components of the medical device may also collide with an object or a person in an examination room, which increases a safety risk in the medical procedure. Therefore, an effective system and method for collision detection need to be provided.

As described in FIG. 3, before or during the execution of the medical procedure, the processing device 130 may detect a control instruction issued by a user for controlling the medical device. For example, the control instruction includes a control instruction "scan a head position of a patient" for scanning the target region of a target object. As another example, the control instruction includes a control instruction "move the C-arm to a spider position," "left angulation of the C-arm 45°," for moving one or more components of the medical device, etc.

In response to the control instruction, the processing device 130 may control one or more components of the medical device to move to a target position to scan the target region of the target object. The target position refers to a position to which one or more components of the medical device need to be moved to. The target region refers to an organ and/or tissue of the target object that needs to be scanned for imaging. For example, when the control instruction is "scan a head position of a patient," the processing device 130 first determines whether the head position of the patient is at a vision center (or a scan center) of the medical device. If the head position of the patient is at the vision center of the medical device, the processing device 130 may control the medical device to scan the head position of the patient. If the head position of the patient is not at the field of view center of the medical device, the processing device 130 can control a component of the medical device (e.g., a C-arm gantry) to move, such that the head position of the patient is located at the field of view center of the medical device, and then scan the head position of the patient.

Merely by way of example, when the control instruction is a control instruction for scanning the target region of the target object, the processing device 130 obtains a third real-time optical image related to the medical device and the target object captured by an image obtaining device installed in the examination room, and determines planned motion information of the one or more components of the medical device based on the third real-time optical image. Further, the processing device 130 may control the one or more components of the medical device to move based on the planned motion information to perform the medical procedure on the target region.

The planned motion information refers to motion information of each component determined in advance before the control instruction is executed. The motion information includes a motion parameter involved by each component during a motion process. For example, the motion parameter includes a type, a motion angle, a motion distance, a motion time, and a motion speed, etc. of a moving component (e.g., a robotic forearm, a robotic upper arm, a C-arm gantry, and a bed plate, etc.). For example, the processing device 130 generates a three-dimensional (3D) object model of the target object based on the third real-time optical image, and determines a position of the target region of the target object based on the 3D object model. Then, the processing device 130 may obtain current position(s) of component(s) of the medical device (e.g., through the parameter of the medical device or the third real-time optical image). The processing device 130 may determine the planned motion information of the component(s) of the medical device based on the position of the target region and the current position(s) of the component(s) of the medical device. Further, the processing device 130 may determine a type of the target region to be scanned (e.g., a head, a liver, etc.) based on the control instruction; determine position information of the target region (e.g., 3D coordinate information of a center point of the head) based on the type of the target region and the 3D object model of the target object; determine the planned motion information of the one or more components of the medical device based on the position information of the target region; and control the one or more components of the medical device to move based on the planned motion information. As another example, the processing device 130 determines the motion parameter of one or more of a patient bed, a detector, a radiation source, etc., so that an imaging isocenter of the medical device is aligned with the center point of the target region.

In some embodiments, before controlling the component(s) to move based on the planned motion information, the processing device 130 performs a virtual collision detection to verify the planned motion information. Specifically, if no collision occurs in the virtual collision detection, the processing device 130 may control the component(s) to move based on the planned motion information. If the collision occurs in the virtual collision detection, the processing device 130 may update the planned motion information until the updated planned motion information passes the virtual collision detection. More descriptions regarding the virtual collision detection may be found below. By performing the virtual collision detection on the planned motion information, a potential collision risk during motion of the component(s) may be determined in advance, thereby eliminating the potential collision risk and improving a safety of the medical procedure.

During motion of the component(s) of the medical device, the processing device 130 may execute a process 700 to perform the collision detection.

In operation 710, during motion of a component of the medical device, the processing device 130 (e.g., the obtaining module 210) obtains a first real-time optical image related to the medical device captured by the image obtaining device.

The first real-time optical image refers to an image related to the medical device (e.g., the medical device 110, the X-ray imaging device) and other objects in the examination room (e.g., the target object) during motion of the component of the medical device, which reflects a real-time state of each object in the examination room.

The first real-time optical image may be captured by the image obtaining device (e.g., the image obtaining device 160) installed in the examination room. More descriptions regarding the image obtaining device may be found in FIG. 1A and related descriptions thereof.

In operation 720, the processing device 130 (e.g., the determination module 230) determines whether the collision occurs during motion of the component based on the first real-time optical image.

For example, the processing device 130 determines a relative position (e.g., a closest distance) between the component and other component(s) or an external object (e.g., the voice device 150, the display device 170, at least one user, the target object, etc.) based on the first real-time optical image. If the relative position is less than a fourth distance threshold, the processing device 130 determines that the collision may occur during motion of the component.

As another example, the processing device 130 determines whether a motion trajectory of the component overlaps with other component(s) or the external object based on the first real-time optical image. If the motion trajectory overlaps with other component(s) or the external object, the processing device 130 may determine that the collision occurs during motion of the component.

In some embodiments, the processing device 130 also determines whether the collision occurs during motion of the one or more components of the medical device in other manners. Merely by way of example, the processing device 130 determines current motion information based on the first real-time optical image, and performs the virtual collision detection based on the current motion information. The current motion information refers to motion information of each component during the execution of the control instruction (e.g., when capturing the first real-time optical image). For example, the processing device 130 generates a virtual imaging system based on feature information of the one or more components of the medical device and/or the real-time optical image. The virtual imaging system includes a virtual representation of each component (e.g., the detector, the radiation source, the gantry, etc.) of the medical device. The virtual imaging system may be used to perform the virtual collision detection. For example, a motion trajectory of the virtual representation corresponding to each component is calculated based on the current motion information of each component, and whether the motion trajectory of the virtual representation corresponding to each component collides with virtual representation(s) of other component(s) is determined. Taking the detector and the patient bed as an example, the processing device 130 may determine whether a current distance between the detector and the patient bed is less than a fourth distance threshold based on positions of a detector model and a patient bed model in the virtual imaging system. If the current distance is less than the fourth distance threshold, it is determined that a collision occurs at a current moment. As another example, the processing device 130 predicts the distance between the detector and the patient bed at the next moment or a time period based on the detector model and the patient bed model in the virtual imaging system, and determines whether the distance is less than the fourth threshold. If the distance is less than the fourth distance threshold, it is determined that there is a collision risk in the future. In some embodiments, the virtual collision detection is established based on a twin digital system. For example, the twin digital system displays, in real time on the display device, the virtual representation of each component (e.g., the detector, the radiation source, the gantry, etc.) of the medical device, the virtual representation of the user in the examination room, etc. It should be understood that, in some embodiments, the processing device 130 may also directly identify the detector and the patient bed from the real-time optical image and perform a distance determination.

As another example, one or more components (e.g., a robotic forearm, a robotic upper arm, a conduit package, an outer arc of the C-arm, a bed base, a flat panel detector, a tube, etc.) of the medical device are mounted with pressure sensors. When the collision occurs at a part mounted with the pressure sensor and a pressure of the collision is greater than a pressure threshold, the processing device 130 determines that the part is collided. The pressure threshold may be a default value, a preset value, etc.

In some embodiments, to improve the safety of one or more components of the medical device during motion, when the processing device 130 detects that a component of the medical device is about to collide with an object or a person during motion, the processing device 130 reduces a motion speed of the component.

If it is determined that no collision occurs during motion of the component, the processing device 130 may control the motion of one or more components of the medical device based on the motion information. If it is determined that the collision occurs during the motion of the one or more components, the processing device 130 may perform operation 730.

In operation 730, the processing device 130 (e.g., the control module 240) controls the first voice device to issue a collision warning.

For example, the processing device 130 sends an instruction for playing the collision warning to the first voice device, to control the first voice device to issue the collision warning. For example, a voice broadcast may be "the robotic forearm end may collide with an infusion stand."

In some embodiments, the processing device 130 also displays a virtual collision warning on the display device. For example, the processing device 130 displays a part that is about to collide and/or has collided on the display device 170. The display device 170 may represent the part that is about to collide and/or has collided with a special identifier. For example, the display device 170 frames the part that is about to collide and/or has collided with a red box. As another example, the display device 170 marks the part that is about to collide and/or has collided with a red exclamation mark. In some embodiments, the processing device 130 determines possible collision events with different urgency levels (e.g., corresponding to different collision probabilities, different collision severities), and represents these collision events with different special identifiers. For example, components involved in the collision events of different urgency levels are marked with boxes of different colors.

In some embodiments, the processing device 130 also issues alert information on a wearable device worn by the target user.

In some embodiments of the present disclosure, not only the part that is about to collide and/or has collided is displayed on the display device, but also the collision warning is broadcasted via the first voice device, thereby providing the user with multi-dimensional prompts of the collision part, and allowing the user to detect the collision risk in time and make a judgment quickly.

In operation 740, the processing device 130 (e.g., the obtaining module 210) obtains a third voice signal.

The third voice signal is generated by the first voice device by detecting a third user voice.

In some embodiments, after the first voice device issues the collision warning, the first voice device detects the third user voice of the user (e.g., the target user), thereby generating the third voice signal. For example, the third user voice includes "ignore the collision warning," "perform virtual collision detection again," "move the robotic arm to the right by 20 centimeters," etc.

In operation 750, the processing device 130 (e.g., the determination module 230) determines a collision response strategy based on the third voice signal.

For example, the processing device 130 determines the collision response strategy based on the control instruction in the third voice signal. For example, in response to the third target voice signal being "ignore the collision warning," the processing device 130 continues to execute the motion control instruction. As another example, in response to the third target voice signal being "move the robotic arm to the right by 20 centimeters," the processing device 130 controls the robotic arm to move to the right by 20 centimeters.

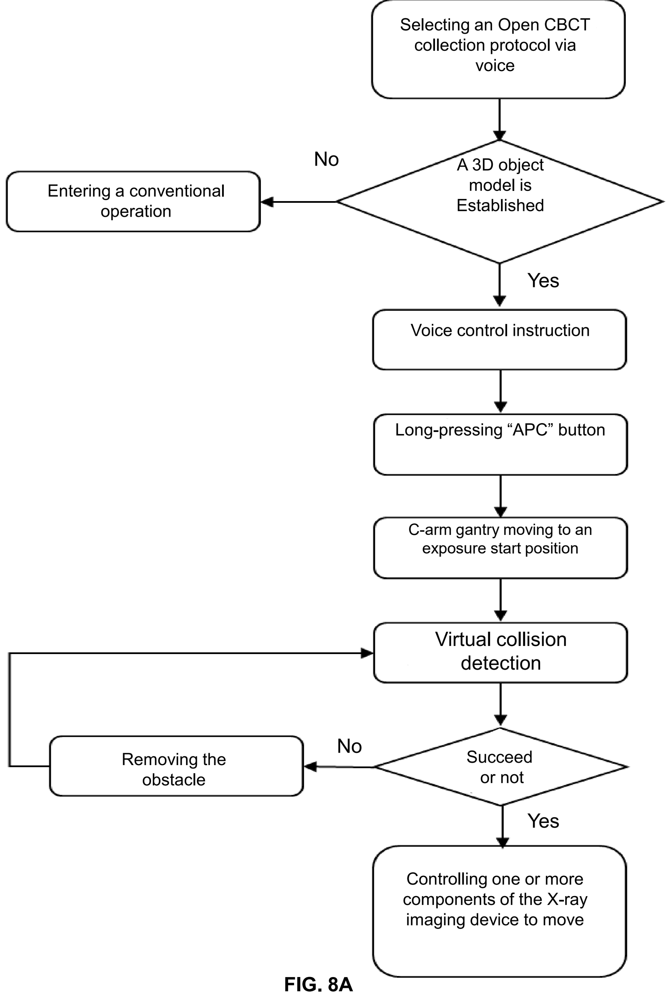

Merely by way of example, as shown in FIG. 8A, the user first selects an Open CBCT collection protocol via voice, and the processing device 130 determines whether the 3D object model of the target object is established. If the 3D object model of the target object is not established, a conventional operation is entered, and the user manually controls the motion of one or more components of the X-ray imaging device. If the 3D object model is established, the processing device 130 obtains the control instruction input by the target user via voice, for example, "scan a liver position of the patient." When the user long-presses an "APC" button, the processing device 130 first controls a C-arm gantry to move to an exposure start position (i.e., automatically placing the liver position at the vision center), then determines the motion parameter based on the 3D object model of the target object and the control instruction, and performs the virtual collision detection based on the motion parameter. If it is detected that the collision may occur, the virtual collision warning is performed via the display device and/or the first voice device, and the virtual collision detection is performed again after an obstacle is removed. If no collision is detected, one or more components of the X-ray imaging device are controlled to move to perform scan imaging on the target region of the target object. In some embodiments, the processing device 130 also performs the virtual collision detection before or during a process of controlling the C-arm gantry to move to the exposure start position. For example, the processing device 130 determines the planned motion information of the C-arm gantry based on the current position of the C-arm gantry and the exposure start position, and performs the virtual collision detection based on the planned motion information.

Merely by way of example, when the user inputs the control instruction "scan a liver position of the patient" via voice, the processing device 130 obtains the control instruction via the voice recognition. In response to the control instruction, the processing device 130 may obtain the real-time optical image related to the medical device and the target object captured by the image obtaining device installed in the examination room, model the target object and the medical device based on the real-time optical image to generate the 3D object model of the target object, and determine a position (e.g., the center point) of the target region of the target object based on the 3D object model. Then, the processing device 130 may obtain the current position of the one or more components of the medical device (e.g., via the parameter of the medical device or the real-time optical image), and determine the planned motion information for moving the vision center of the medical device to the exposure start position (i.e., placing the liver position at the vision center) based on the position of the target region and the current position of the one or more components of the medical device. In some embodiments, the processing device 130 performs the virtual collision detection based on the planned motion information. After the liver position is placed at the vision center of the medical device, the processing device 130 may control the medical device to perform a scan operation.

In some embodiments, when the control instruction is the control instruction for moving the one or more components of the X-ray imaging device, the storage device 140 pre-stores a correspondence between different control instructions and motion parameters. After the first voice device receives the control instruction input by the target user, the first voice device sends the control instruction to the processing device 130. The processing device 130 may access the storage device 140 based on the determined control instruction, determine the motion information via the correspondence, and control the motion of the one or more components of the X-ray imaging device based on the motion information. In some embodiments, the processing device 130 processes the control instruction using a voice processing model to obtain a semantic text corresponding to the control instruction, and determines the motion information based on the semantic text. In some embodiments, the voice processing model includes a large language model (LLM), which includes various deep learning models trained using a large amount of text data.

In some embodiments, to improve safety of motion of the one or more components of the X-ray imaging device, the processing device 130 recognizes an identity of at least one user based on the control instruction of the at least one user, and determines whether the at least one user includes the target user (e.g., an operator of the X-ray imaging device). If the at least one user includes the target user, the processing device 130 executes the control instruction input by the target user; if the at least one user does not include the target user, the processing device 130 determines whether the user has a control permission, and if the user does not have a control permission, the processing device 130 issues a prompt via the first voice device (e.g., by playing a voice message "No control permission").

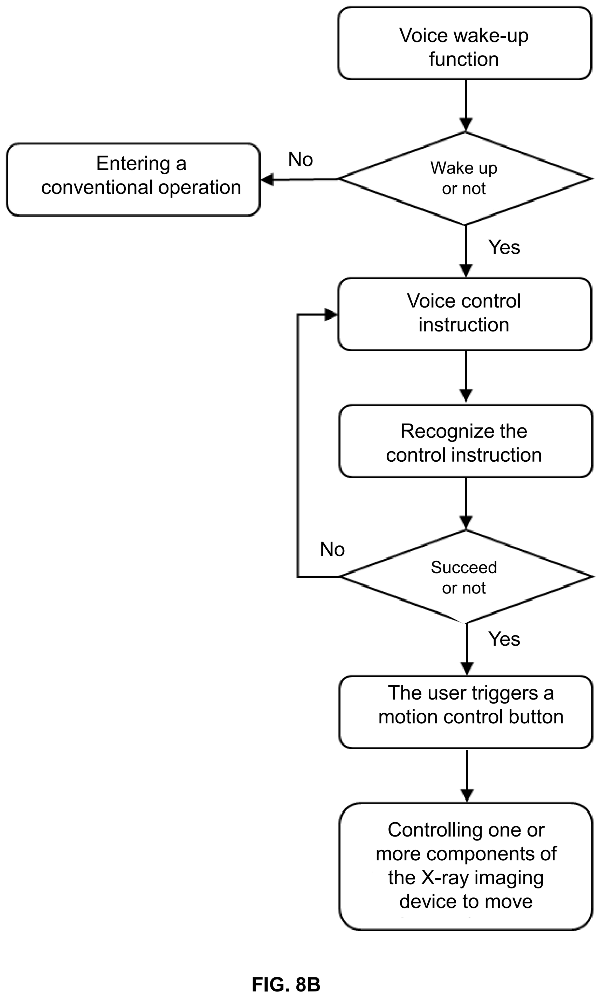

As another example, as shown in FIG. 8B, the user starts a voice control of the X-ray imaging device through a voice wake-up function. If the wake-up is failed, the system enters a conventional operation mode, and the user manually controls the motion of the one or more components of the X-ray imaging device. If wake-up is successfully initiated, the processing device 130 obtains the control instruction input by the user via the first voice device, for example, "C-arm left angulation 45°." The processing device 130 may recognize the control instruction. Recognizing the control instruction refers to processing the control instruction to determine a text corresponding to the control instruction and determining the motion parameters based on the text. If the corresponding text can be recognized and the motion information can be determined based on the text, the recognition is successful; if the corresponding text cannot be recognized or the motion information cannot be determined based on the text, the recognition is failed. If the processing device 130 cannot recognize the control instruction, the processing device 130 prompts the user to re-enter the control instruction via the first voice device. If the processing device 130 successfully recognizes the control instruction, when the user triggers a motion control button, the processing device 130 controls the motion of one or more components of the X-ray imaging device based on the motion information corresponding to the control instruction.

Merely by way of example, taking a common percutaneous coronary intervention (PCI) procedure as an example, during the procedure, in order to better observe lesions in the left and right coronary arteries of the patient, the C-arm needs to be frequently switched among 8 clinical angles, leading to problems such as a prolonged positioning time and an increased ineffective radiation exposure to the patient and doctor. By controlling the C-arm to quickly and accurately reach a target position through the voice control instruction, the user can not only achieve more precise treatment but also significantly improve the positioning efficiency of the C-arm during the procedure and reduce a radiation damage.

In some embodiments of the present disclosure, by enabling one or more components of a medical device to move quickly and accurately to a target position through the voice control instruction, not only the doctor's observation of the examination condition of the target region of the patient can be facilitated from different angles, thereby improving a detection accuracy, but also the positioning efficiency of the one or more components of the medical device can be significantly enhanced during the medical procedure, thereby reducing a radiation damage caused by a plurality of adjustments of the positioning angles of the components due to manual operational errors.

In some embodiments, in order to improve safety of the one or more components of the medical device during motion and avoid collisions among the components and other objects or persons during motion, after the processing device 130 receives the control instruction input by the user and determines the motion information, the processing device 130 enters a waiting-for-motion-trigger state; when the user further confirms the motion instruction, the processing device 130 sends the motion information to the medical device to control the one or more components of the medical device to move to the target position.

There are various manners for the user to further confirm the motion instruction. For example, the user further confirms the motion instruction by triggering a motion control button. As another example, the user further confirms the motion instruction by voice inputting "Confirm motion" via the first voice device.

In some embodiments, during the motion of the one or more components, the processing device 130 updates the real-time optical image and performs the collision warning based on updated real-time optical image. For example, the processing device 130 repeats operations 710-750 until the one or more components of the medical device move to the target position.

In some embodiments of the present disclosure, by detecting whether the collision occurs during motion of the one or more components of the medical device using the updated real-time optical image and/or a pressure sensor, moving collision issues are identified promptly, thereby improving safety during the medical procedure.

In some embodiments of the present disclosure, by using the real-time optical image and the voice control instruction to control the medical device to scan the target region of the patient and detect the collision issues during motion, not only an automatic control of motion of the one or more components of the medical device can be achieved, thereby enabling a quick and accurate arrival at the target position for scanning and imaging the target region and reducing a dose of ineffective radiation, but also the moving collision issues can be effectively avoided, thereby improving safety during the procedure. Furthermore, by combining an advantage of a wide monitoring range of an optical anti-collision and an advantage of convenience and simplicity of the voice control, the accuracy and efficiency of intelligent control can be further improved.

In some embodiments, process 700 further includes one or more other operations.

For example, before the first voice signal is detected, the processing device 130 (e.g., the control module 240) obtains a second real-time optical image related to the medical device captured by the image obtaining device installed in the examination room. The second real-time optical image is captured by the image obtaining device (e.g., the image obtaining device 160) installed in the examination room, and the second real-time optical image reflects states of various objects in the examination room before the first voice signal is detected. For example, the second real-time optical image displays the position information of the target object to be scanned and the one or more components of the medical device.

Further, the processing device 130 may control the display device to display a target interface based on the second real-time optical image. The target interface refers to an interface for reflecting a real-time state of the target object and the medical device. The real-time state of the target object and the medical device may include a positional relationship between the target object and the medical device, for example, a minimum distance between the target object and the medical device. For example, the second real-time optical image is displayed in the target interface. As another example, the processing device 130 reconstructs the second real-time optical image to obtain one or more of a 3D object model of the target object, a 3D device model of the medical device, 3D body models of other persons (e.g., doctors) or objects (e.g., the voice device 150, the display device 170, an operating lamp) in the examination room, etc., and displays these 3D models in the target interface.

In some embodiments, before the first voice signal is detected, the second real-time optical image is continuously updated; correspondingly, the target interface (e.g., the real-time state of the target object and the medical device) is continuously updated, allowing the user to understand real-time dynamics of the examination room.

During the process in which the user controls the motion of the one or more components of the medical device via voice, the processing device 130 may update the target interface based on the first real-time optical image. For example, the processing device 130 presents the first real-time optical image in the target interface. Alternatively, the processing device 130 may reconstruct the first real-time optical image to obtain one or more of the 3D object model of the target object, the 3D device model of the medical device, the 3D body models of other persons (e.g., doctors) or objects (e.g., the voice device 150, the display device 170, the operating lamp) in the examination room, etc., and sends these 3D models to the display device 170 for display or update the target interface. When it is detected that the collision may occur, the processing device 130 may control the display device to present the collision warning in the target interface.

FIG. 9 is a schematic diagram illustrating an exemplary intelligent control system according to some embodiments of the present disclosure. An intelligent control system 900 is a variation of the intelligent control system 100. Some components of the intelligent control system 900 have the same or similar functions and/or structures as corresponding components of the intelligent control system 100.

As shown in FIG. 9, the intelligent control system 900 may include the processing device 130, a network 920, a system control device 930, a first voice device 940, and a second voice device 950. Different components of the intelligent control system 900 may be arranged in different rooms.

The processing device 130 may process data and/or information obtained from the system control device 930, the first voice device 940, and the second voice device 950. For example, the processing device 130 processes voice signals obtained from the first voice device 940 and the second voice device 950. In some embodiments, the processing device 130 is communicatively connected to the first voice device 940 and the second voice device 950 respectively, and thus obtains voice signals collected by the first voice device 940 and/or the second voice device 950.

In some embodiments, the processing device 130 processes the voice signal. For example, the processing device 130 performs a voiceprint recognition on the voice signal to determine identity information of a speaker (e.g., a doctor, a technician, etc.). As another example, the processing device 130 performs a voice recognition on a voice signal (e.g., the first voice signal) to determine whether the voice signal includes a control instruction related to a medical device. In some embodiments, the processing device 130 sends the control instruction related to the medical device (e.g., an X-ray imaging device, a DSA device, etc.) to the system control device 930, so that the system control device 930 executes one or more control operations related to the medical device in a medical procedure (e.g., an X-ray imaging, a DSA examination, etc.). More information about the voice recognition may be found in FIG. 3 and the related descriptions. In some embodiments, the processing device 130 extracts a target voice signal corresponding to a target user from the voice signal (e.g., the first voice signal). More descriptions regarding extracting the target voice signal may be found in FIG. 4 and the related descriptions.

As shown in FIG. 9, the processing device 130 may be deployed in a device room. The device room refers to a room for deploying various devices including but not limited to the processing device 130 and the system control device 930. In some embodiments, the processing device 130 is deployed in a control room or an examination room. In some embodiments, the processing device 130 or a portion thereof may be deployed in a combination of the device room, the control room, and the examination room in a distributed manner. In some embodiments, the processing device 130 is remote. For example, the processing device 130 is implemented on a cloud platform.

The network 920 may include any suitable network that facilitates information and/or data exchange of the intelligent control system 900. In some embodiments, one or more components (e.g., the processing device 130, the system control device 930, the first voice device 940, and the second voice device 950) of the intelligent control system 900 transmits information and/or data to one or more other components of the intelligent control system 900 via the network 920. In some embodiments, the network 920 is an Ethernet, which is implemented via a network switching device. The network switching device may be configured to implement a communication connection between the processing device 130 and the first voice device 940, and a communication connection between the processing device 130 and the second voice device 950.

In some embodiments, the network switching device (not shown in the figure) is one or more network data exchange/forwarding devices, which is deployed in the device room or other suitable places (e.g., the control room, the examination room, etc.). For example, the network switching device is a network switch, a router, or a combination thereof. The network switching device may include one or more access points for implementing connections and data exchange among one or more other components (e.g., the first voice device 940, the second voice device 950, etc.) of the intelligent control system 900.

In some embodiments, the network switching device is used to implement a voice interaction between the first voice device 940 and the second voice device 950. The voice interaction process is referred to as a voice intercom. Merely by way of example, the voice signal of the first voice device 940 is transmitted to the processing device 130 via the network switching device. Further, the processing device 130 transmits the voice signal to the second voice device 950 via the network switching device, so that the second voice device 950 plays the voice signal through a voice output component (e.g., a speaker) of the second voice device 950, thereby implementing a voice intercom process from the first voice device 940 to the second voice device 950.

Similarly, the voice signal of the second voice device 950 may be transmitted to the processing device 130 via the network switching device. Further, the processing device 130 may transmit the voice signal to the first voice device 940 via the network switching device, so that the first voice device 940 plays the voice signal through the voice output component (e.g., the speaker) of the first voice device 940. Thereby, the voice intercom process from the second voice device 950 to the first voice device 940 is implemented.

The system control device 930 refers to a device for controlling a medical device. More descriptions regarding the system control device may be found in FIG. 1A and the related descriptions.

The voice device may be used to implement a voice interaction among a plurality of users. The first voice device 940 refers to a voice device deployed in the examination room. The second voice device 950 refers to a voice device deployed in the control room. The control room is a place where a control worker of the medical procedure is located. The control worker may issue control instructions related to the medical procedure (e.g., a control instruction for the medical device) in the control room, observe and monitor the execution process of the medical procedure, and provide guidance to a patient or other users in the examination room, etc. The examination room is a place where the medical procedure is performed. The medical device (e.g., a CT device, a DSA device, a radiotherapy device, etc.) may be deployed in the examination room. In addition, the patient undergoing the medical procedure, the technician performing the medical procedure, etc., may be located in the examination room.

The worker in the control room and the worker in the examination room may perform the voice intercom via the first voice device 940 and the second voice device 950. Hereinafter, one or more workers in the examination room may be referred to as a first type of user, and one or more workers in the control room may be referred to as a second type of user. The first type of user and the second type of user may include the doctor, the technician, or other medical workers. The first type of user may also include the patient. For example, during a scan, the second type of user may be the doctor, and the first type of user may be a scanning technician and the patient.

The voice device may also be used to implement the voice control of the device. For example, the first type of user and/or the second type of user issue a voice control instruction via a corresponding voice device to perform an intelligent control of devices in the examination room, the control room, and the device room. Merely by way of example, the doctor in the examination room controls the medical device in the examination room and the display device in the control room via voice. Therefore, through the intelligent control system 900 including the processing device 130 and the voice devices (e.g., the first voice device 940 and the second voice device 950), an interaction between the user and the target device (e.g., the medical device, the display device, etc.) may be implemented. For example, the processing device 130 performs the voice recognition on the voice collected by the voice device to obtain the control instruction, and controls the target device through the control instruction.

Hereinafter, a voice of the first type of user is referred to as a first user voice, and a signal related to the first voice is referred to as a first voice signal. A voice of the second type of user is referred to as a third user voice, and a signal related to the third voice is referred to as a third voice signal.

As shown in FIG. 9, the first voice device 940 may include a first voice input component 941, a first voice output component 942, and a first voice processing component 943. The second voice device 950 may include a second voice input component 951, a second voice output component 952, and a second voice processing component 953.

The voice input component may be used to collect or detect the user voice. The voice input component may include various sound collection devices such as a microphone, a voice recorder, etc.

The voice output component may be used to play a sound. The voice output component may include various sound output devices such as the speaker, a sound device, a horn, etc.

The voice processing component may be used to perform a voice signal conversion and processing. In some embodiments, the voice processing component includes an audio signal codec for encoding the user voice (i.e., an analog signal) detected by the voice input component into the voice signal (i.e., a digital signal) and transmitting the voice signal to the processing device 130 for voice recognition or other processing, or for decoding the voice signal into an analog signal for playback via the voice output component, etc. In some embodiments, the voice processing component further includes various components related to sound processing, such as a filter, a noise reducer, etc.

Taking the first voice device 940 as an example, the first voice input component 941 is configured to detect the first voice of the first type of user. The first voice output component 942 is configured to play the second voice of the second type of user. The first voice processing component 943 may be configured to convert the first voice into the first voice signal, convert the third voice signal into a playable second voice, and perform a process operation (e.g., a noise reduction, a sound amplification, etc.) on the first voice signal and/or the third voice signal.

In some embodiments, the voice input component and the voice output component in each voice device are respectively connected to the voice processing component of the voice device. The voice processing components of the two voice devices are further connected to the network 920.

In some embodiments, the intelligent control system 900 further includes other devices, such as the medical device 110, the storage device 140, the image obtaining device 160, the display device 170, etc. More descriptions regarding the medical device, the storage device, the image obtaining device, and the display device may be found in FIG. 1A and related descriptions.

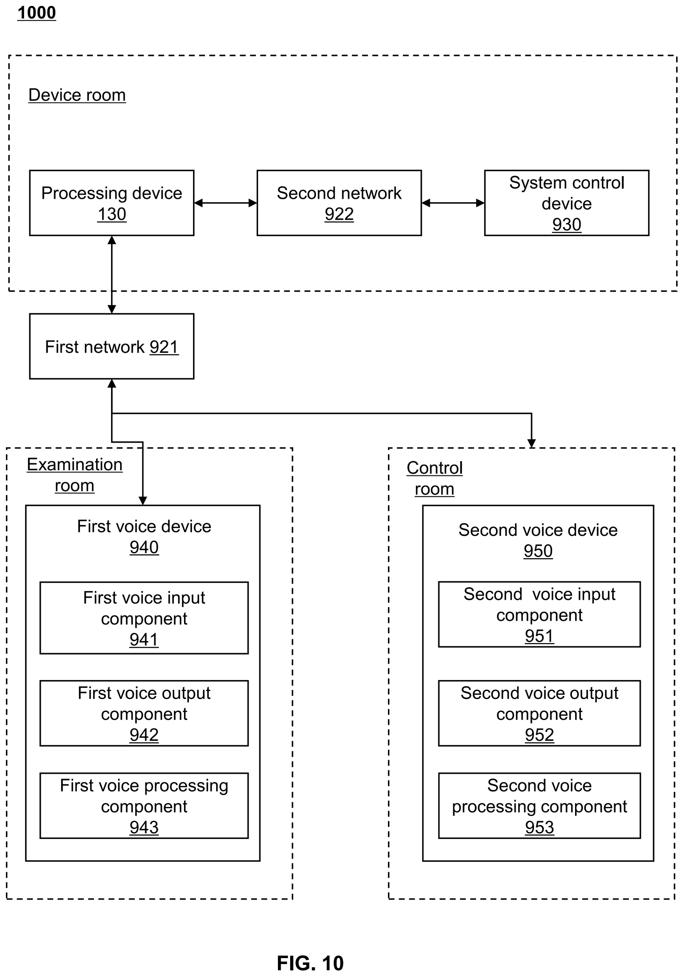

FIG. 10 is a schematic diagram illustrating an exemplary intelligent control system according to some embodiments of the present disclosure.

The intelligent control system 1000 is similar to the intelligent control system 900. A difference is that the intelligent control system 1000 includes a first network 921 and a second network 922. The first network 921 is configured to implement data communication between the processing device 130 and the first voice device 940 and the second voice device 950. The second network 922 is configured to implement data communication between the processing device 130 and the system control device 930. In some embodiments, the first network 921 is connected to the first voice processing component 943 and the second voice processing component 953.

In some embodiments of the present disclosure, using the first network 921 to transmit data related to a voice intercom scenario and using the second network 922 to transmit data related to a control scenario of the medical device can avoid a mutual interference between the two types of data transmission, which ensures a sufficient transmission bandwidth for each type of data and improves data transmission efficiency.

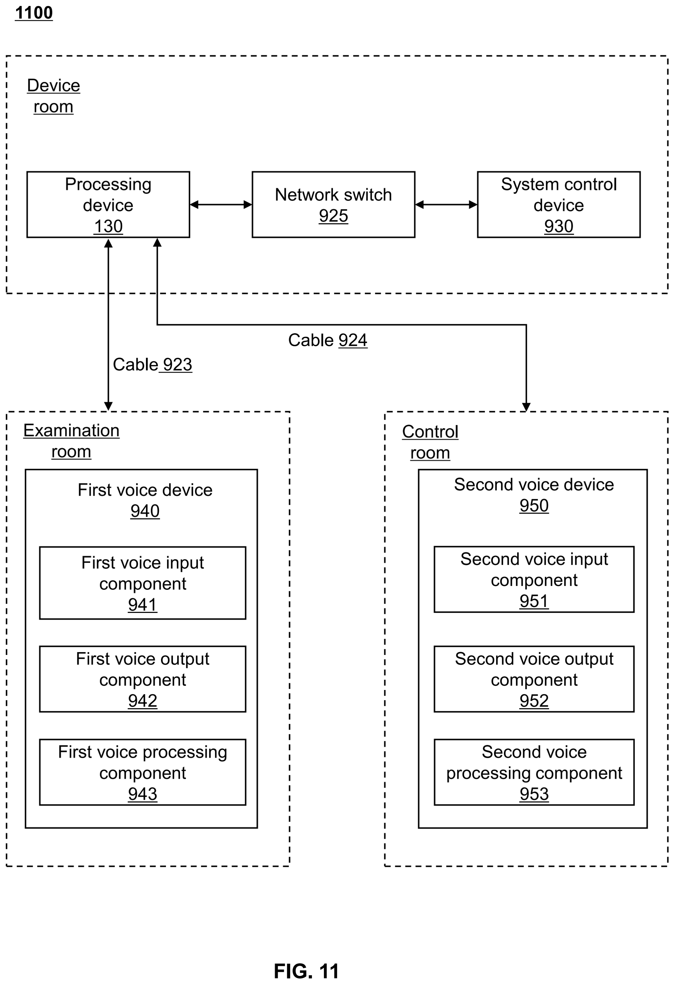

The first network 921 and the second network 922 may be the same type or different types of networks. Merely by way of example, an intelligent control system 1100 shown in FIG. 11 is a specific embodiment of the intelligent control system 1000. As shown in FIG. 11, the first network 921 is implemented by a cable 923 and a cable 924. The second network 922 is implemented by a network switch 925.

The cable 923 is configured to implement a communication connection between the processing device 130 and the first voice device 940. The cable 923 may be various types of cables. The cable 923 may be determined based on an interface type of the processing device 130 or the first voice device 940. In some embodiments, the processing device 130 includes various types of interfaces. For example, the processing device 130 includes any one or a combination of a plurality of universal serial bus (USB) interfaces and tip ring sleeve (TRS) audio interfaces. The cable 923 may be a USB cable, a TRS cable, etc., for connecting the first voice device 940 to the processing device 130.

The cable 924 is configured to implement a communication connection between the processing device 130 and the second voice device 950. Similar to the cable 923, the cable 924 may also be various types of cables (e.g., a USB cable).

The network switch 925 refers to a network switching device configured to implement network data forwarding between the processing device 130 and the system control device 930. More descriptions regarding the network switching device may be found in FIG. 9 and the descriptions thereof.

In some embodiments of the present disclosure, by directly connecting the processing device 130 to the first voice device 940 and the second voice device 950 via cables (e.g., the cable 923 and the cable 924), a direct data transmission between the processing device 130 and the first voice device 940 and the second voice device 950 can be achieved, which improves an efficiency of voice intercom between the first voice device 940 and the second voice device 950.

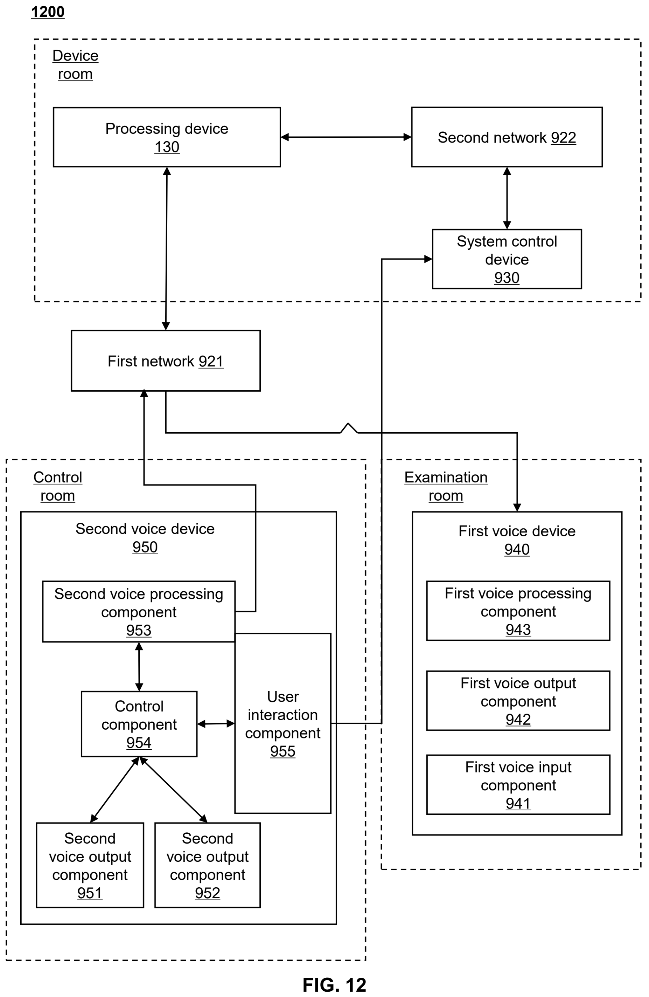

FIG. 12 is a schematic diagram illustrating an exemplary intelligent control system according to some embodiments of the present disclosure.

An intelligent control system 1200 is similar to the intelligent control system 1000 in FIG. 10. A difference is that a structure of the second voice device 950 is different. As shown in FIG. 12, the second voice device 950 may include a second voice input component 951, a second voice output component 952, a second voice processing component 953, a control component 954, and a user interaction component 955. The control component 954 is communicatively connected to the second voice output component 952, the second voice input component 951, the second voice processing component 953, and the user interaction component 955, respectively.

The control component 954 is configured to control an on/off state of the second voice output component 952 and the second voice input component 951. For example, the control component 954 is provided with switch buttons for the second voice input component 951 and the second voice output component 952. A user may turn on or off the second voice input component 951 and the second voice output component 952 via these buttons. In some embodiments, the control component 954 includes a keypad board.

The user interaction component 955 may be configured to send the on/off state of the second voice input component 951 and the second voice output component 952 to the system control device 930. As shown in FIG. 12, the user interaction component 955 is further communicatively connected to the system control device 930 and is configured to send the on/off state of the second voice input component 951 and the second voice output component 952 to the system control device 930. The system control device 930 may send the on/off state to the processing device 130 via the second network 922. The processing device 130 is further configured to control a connection state between the processing device 130 and the second voice device 950 based on the on/off state. Merely by way of example, when the second voice input component 951 or the second voice output component 952 is turned on, the processing device 130 establishes a connection with the second voice device 950 and receives a voice signal collected by the second voice device 950 or sends a voice signal to the second voice device 950. When the second voice input component 951 and the second voice output component 952 are turned off, the processing device 130 may disconnect the connection with the second voice device 950.

In some embodiments, the user interaction component 955 may be communicatively connected to the system control device 930 via a controller area network (CAN) bus.

The foregoing descriptions are for illustrative purposes only. Actual application scenarios may have various changes. For example, the user interaction component 955 is configured to be communicatively connected to the processing device 130 via the second network 922, thereby sending the on/off state of the second voice input component 951 and the second voice output component 952 to the processing device 130. As another example, a structure of the first voice device 940 is similar to a structure of the second voice device 950, which also includes a control component and a user interaction component. Specifically, the control component of the first voice device 940 may be communicatively connected to the voice output component 942 and the voice input component 941, and may be configured to control the on/off state of the voice output component 942 and the voice input component 941. The user interaction component of the first voice device 940 may be communicatively connected to the control component of the first voice device 940 and the processing device 130, and may be configured to send the on/off state of the voice output component 942 and the voice input component 941 to the processing device 130. Therefore, the processing device 130 may be further configured to control the connection state between the processing device 130 and the first voice device 940 based on the on/off state of the voice output component 942 and the voice input component 941.

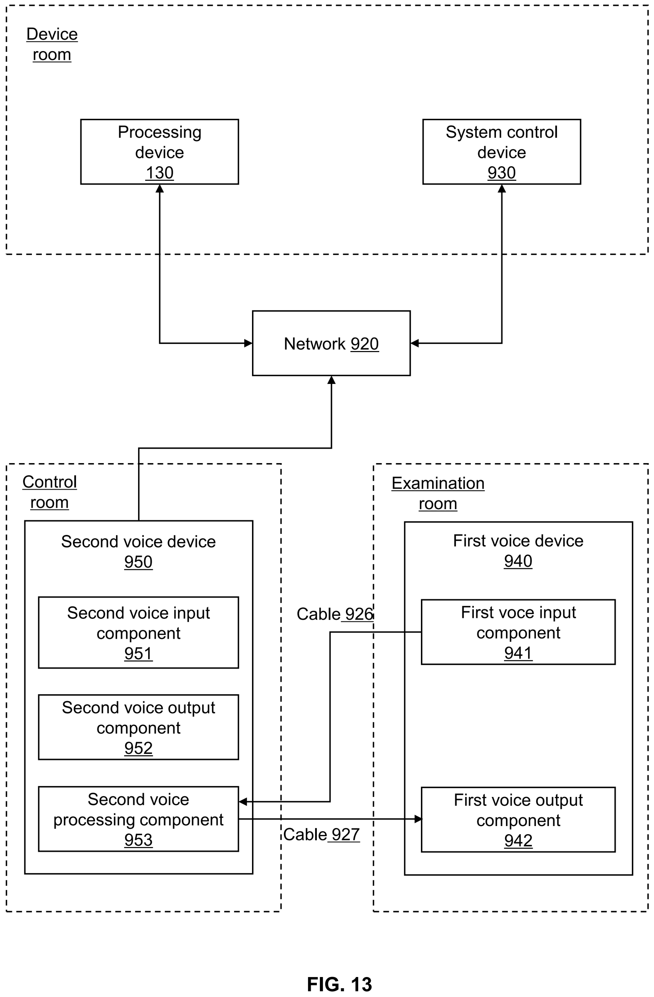

FIG. 13 is a schematic diagram illustrating an exemplary intelligent control system according to some embodiments of the present disclosure.

As shown in FIG. 13, an intelligent control system 1300 may include the processing device 130, the network 920, the system control device 930, the first voice device 940, and the second voice device 950. The processing device 130 and the system control device 930 may be disposed in a device room or other suitable places (e.g., a control room). The first voice device 940 is disposed in an examination room. The second voice device 950 is disposed in the control room. The second voice device 950 includes the second voice input component 951, the second voice output component 952, and the second voice processing component 953. The first voice device 940 includes the first voice input component 941 and the first voice output component 942 but does not include a voice processing component.

The processing device 130, the network 920, the system control device 930, and the second voice device 950 may be similar to the processing device 130, the network 920, the system control device 930, and the second voice device 940 respectively in the intelligent control system 900 shown in FIG. 9, which are not repeated herein.

The second voice device 950 is communicatively connected to the processing device 130. The first voice device 940 is communicatively connected to the second voice device 950. For example, as shown in FIG. 13, the second voice device 950 is communicatively connected to the processing device 130 via the network 920. The first voice input component 941 and the first voice output component 942 in the first voice device 940 may be communicatively connected to the second voice processing component 953 in the second voice device 950 via the cable 926 and the cable 927, respectively.

In some embodiments, when a distance between the control room and the examination room is less than a preset distance threshold, the method shown in FIG. 13 is used to directly communicatively connect the first voice device 940 to the second voice device 950. The preset distance threshold may be 20 m or other suitable values.

In some embodiments, the first voice device 940 is regarded as a part of the second voice device 950. For example, when building the intelligent control system 1300, one of a plurality of microphones of the second voice device 950 is placed in the examination room as the first voice input component 941. One of a plurality of speakers of the second voice device 950 is placed in the examination room as the first voice output component 942.

In some embodiments, the second voice device 950 is configured to detect a third user voice to generate a third voice signal, and to send the third voice signal to the processing device 130. The processing device 130 is configured to send the third voice signal to the first voice device 940. The first voice device 940 is configured to convert the third voice signal into a user voice and output the user voice. The first voice device 940 is further configured to detect a first user voice and send the detected first user voice to the second voice device 950. The second voice device 950 is configured to convert the first voice signal into the user voice and output the user voice. In such embodiments, the first voice device 940 is only used for sound collection and sound output, and a voice processing (e.g., a noise reduction processing) is performed by the second voice processing component 953 of the second voice device 950.

The first user voice refers to a user voice corresponding to one or more users in the examination room. The first voice signal is a voice signal corresponding to the first user voice. The third user voice refers to a user voice corresponding to one or more users in the control room. The third voice signal is a voice signal corresponding to the third user voice. More descriptions regarding the user voice and the voice signal may be found in FIG. 3 and the descriptions thereof.

In some embodiments, the system control device 930 is used to execute a preset control instruction related to a medical device. The system control device 930 is communicatively connected to the processing device 130.

In some embodiments, the processing device 130 is further configured to perform a voice recognition on the first voice signal to obtain a voice recognition result. Based on the voice recognition result, the processing device 130 determines whether the first voice signal includes a control instruction. In response to determining that the first voice signal includes the control instruction, the processing device 130 sends the voice recognition result to the system control device 930, so that the system control device 930 controls the medical device. More descriptions regarding the voice recognition result and the control instruction may be found in FIG. 15 and the related descriptions, which are not repeated here.

In some embodiments, the processing device 130 extracts a target voice signal emitted by a target user from the first voice signal and send the target voice signal to the second voice device 950. More descriptions regarding the extraction of the target voice signal may be found in FIG. 4 and the descriptions thereof, which are not repeated here.

In some embodiments of the present disclosure, the voice input component (e.g., the microphone) and the voice output component (e.g., the speaker) need to be set in the examination room. The voice signal from the examination room is directly received and processed (e.g., a noise reduction processing is performed thereon) by the second voice device 950 via the cable. In this way, a network resource consumption is reduced, and an overall structure of the intelligent control system is made simpler and easier to maintain.

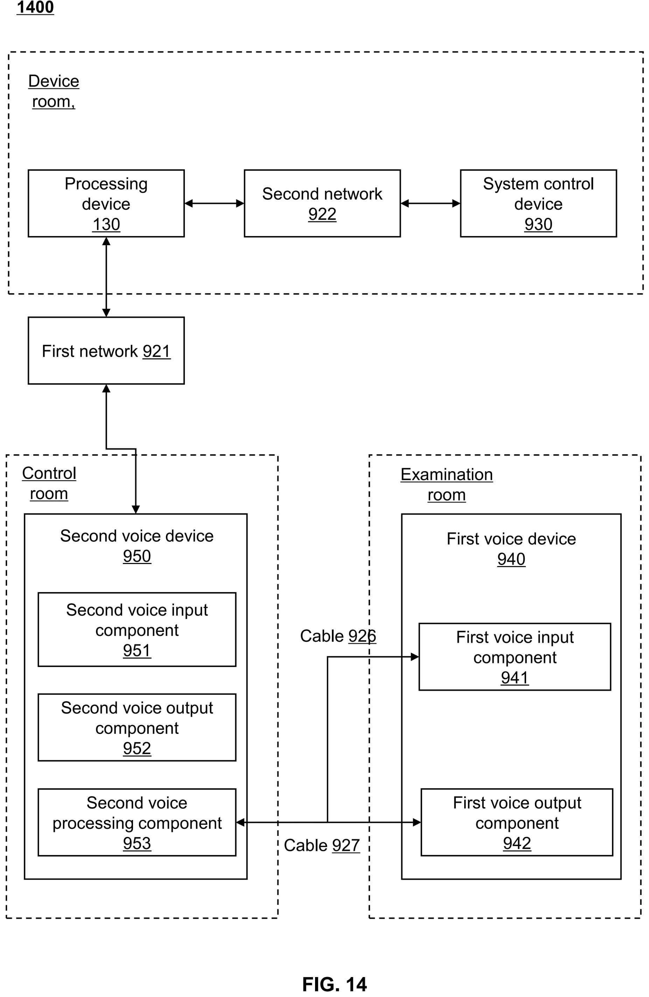

FIG. 14 is a schematic diagram illustrating an exemplary intelligent control system according to some embodiments of the present disclosure.

An intelligent control system 1400 is similar to the intelligent control system 1300. A difference is that the intelligent control system 1400 includes a first network 921 and a second network 922. As shown in FIG. 14, the first network 921 is used to implement a data communication between the processing device 130 and the second voice device 950. The second network 922 is used to implement a data communication between the processing device 130 and the system control device 930.

The above description of the intelligent control system is for illustrative purposes only. An actual intelligent control system may have various changes. For example, a connection manner among various components in the intelligent control system may change. As another example, taking the intelligent control system 1300 as an example, the intelligent control system 1300 further includes a voice signal encoding device and a voice signal decoding device. The voice signal encoding device and the voice signal decoding device may be disposed between the first network 921 and the processing device 130, and are used for voice signal transmission or conversion.

FIG. 15 is a schematic diagram illustrating an exemplary voice interaction process between a first voice device and a second voice device according to some embodiments of the present disclosure. In some embodiments, process 1500 is executed by the processing device 130.

As shown in FIG. 15, the processing device 130 may receive a first voice signal 1502 from the first voice device 940. For example, the processing device 130 obtains the first voice signal 1502 via the network 920 or the first network 921. The processing device 130 may perform a voice recognition on the first voice signal 1502 to obtain a voice recognition result 1504. More descriptions regarding obtaining the voice recognition result may be found in FIG. 4 and the related descriptions. Based on the voice recognition result 1504, the processing device 130 may determine a control instruction 1506 (or a target voice signal) corresponding to the first voice signal 1502, and send the control instruction 1506 (or the target voice signal) to the second voice device 950. More descriptions regarding determining the control instruction may be found in FIG. 3 and the related descriptions. In some embodiments, the second voice device 940 plays the control instruction 1506 (or the target voice signal). In some embodiments, the first voice signal 1502 and the control instruction 1506 (or the target voice signal) are sent to the second voice device 940 together. The second voice device 940 plays the first voice signal 1502.

Similarly, the processing device 130 may receive a third voice signal from the second voice device 950. The processing device 130 may perform a voice recognition on the third voice signal to obtain a voice recognition result. Based on the voice recognition result, the processing device 130 may determine a control instruction (or a target voice signal) corresponding to the third voice signal, and send the control instruction (or the target voice signal) to the first voice device 940.

FIG. 16 is a flowchart illustrating an exemplary process for controlling a medical device according to some embodiments of the present disclosure.

In some embodiments, operation 340 in FIG. 3 may be implemented through process 1600. In some embodiments, process 1600 is executed by the processing device 130.

In operation 1610, the processing device 130 generates a structured control instruction by performing a structured processing on the control instruction.

The structured control instruction has a preset data structure. The preset data structure may be determined based on a medical protocol. Medical protocols corresponding to different medical procedures may define different data structures of the control instructions. For example, the preset data structure includes a medical device type, a component type, and an operation parameter. The medical device type may include a type of the medical device in an examination room (e.g., a CT device, a DSA device), etc. The component type may refer to a target component of the medical device that needs to be operated (e.g., a gantry of the DSA). The operation parameter may include a specific operation on the target component (e.g., a motion distance, a rotation angle, etc.).

In some embodiments, the processing device 130 performs a structured processing on the voice recognition result to determine the structured control instruction.

In operation 1620, the processing device 130 sends the structured control instruction to a system control device, so that the system control device controls the medical device.

The processing device 130 may determine, based on the structured control instruction and a preset control instruction set, whether the control instruction is a target type of control instruction. The preset control instruction set may include one or more preset control instructions related to the medical device. Each preset control instruction in the control instruction set has a preset data structure (i.e., the structured control instruction). For example, the processing device 130 determines whether the medical device type and the component type in the structured control instruction corresponding to the control instruction are consistent with the medical device type and the component type of the preset control instruction. If the medical device type and the component type in the structured control instruction corresponding to the control instruction are inconsistent with the medical device type and the component type of the preset control instruction, it indicates that the control instruction corresponding to the voice recognition result is not the target type of control instruction. If the medical device type and the component type in the structured control instruction corresponding to the control instruction are consistent with the medical device type and the component type of the preset control instruction, it indicates that the control instruction corresponding to the voice recognition result includes the target type of control instruction.

In response to determining that the control instruction is the target type of control instruction, the processing device 130 may send the structured control instruction to the system control device, so that the system control device controls the medical device.

Compared with an original voice recognition result/control instruction, the structured control instruction has more concise and accurate content. Therefore, sending the structured control instruction improves an accuracy and an efficiency of controlling the medical device. In response to determining that the voice recognition result does not include the target type of control instruction, the processing device 130 does not need to send the voice recognition result to the system control device 930, and may generate a notification message and send it to the first voice device 940 and/or the second voice device 950, to inform relevant worker, so that the relevant worker know that a current voice conversation does not generate the control instruction related to the medical device. The relevant worker may reorganize a voice content according to actual needs (e.g., whether a current voice conversation is expected to complete the control of the medical device, or is merely a communication with a worker in the examination room). The notification message may be in various forms, such as a text (e.g., a message presented on the display device), a voice (e.g., feedback played by a speaker of the first voice device 940), etc.

In some embodiments, the processing device 130 extracts a target voice signal from the first voice signal, and then determines whether the target voice signal includes the control instruction of the target type. In this manner, the control instruction for controlling the medical device issued by the target user may be accurately identified, and the medical device may be accurately controlled accordingly.

In some embodiments of the present disclosure, (1) by processing the user's voice signal through the voice recognition, the intelligent voice control of the target device and the automated control of the medical device operations can be achieved, enabling the doctors to interact with the medical device via voice without manually controlling the medical device, thereby reducing a number of medical workers required in the medical procedure, improving the execution efficiency and accuracy of the medical procedure, and thus ensuring that the medical procedure proceeds more smoothly; (2) by analyzing the voiceprint information to identify the identity of the speaking user, and only identifying and processing the target voice signal corresponding to the target user, an impact of non-target users' voices on the control of the medical procedure can be avoided, thereby improving the accuracy and safety of the medical device operation control, and ensuring that the medical procedure proceeds more smoothly; (3) through the intelligent voice control, the display interface of the display device is automatically configured in a personalized manner, and the display parameters of the display interface are adjusted according to the user's voice instructions, thereby improving the execution efficiency of the medical procedure while enhancing the user experience; (4) through the real-time optical images and the voice control instructions, the medical device is controlled to scan the target region of the patient, and the collision issues during motion are detected, not only realizing the automatic control of the motion of the one or more components of the medical device to quickly and accurately reach the target position to scan and image the target region, and reducing the dose of invalid radiation, but also effectively avoiding the collision issues during motion, and improving safety during the surgical procedure.

Some embodiments of the present disclosure further provide a computer-readable storage medium. The storage medium stores computer instructions. When a computer reads the computer instructions, the computer executes the method for intelligent control described in the present disclosure. For more technical details, reference may be made to the relevant descriptions in FIGs. 1A-16, which are not repeated here.

The basic concepts have been described above. Obviously, to those skilled in the art, the above detailed disclosure is merely an example and does not constitute a limitation on the present disclosure. Although not explicitly stated herein, those skilled in the art may make various modifications, improvements, and amendments to the present disclosure. Such modifications, improvements, and amendments are suggested in the present disclosure, so they still fall within the spirit and scope of the exemplary embodiments of the present disclosure.

Meanwhile, the present disclosure uses specific words to describe the embodiments of the present disclosure. For example, "one embodiment," "an embodiment," and/or "some embodiments" mean that a certain feature, structure, or characteristic is related to at least one embodiment of the present disclosure. Therefore, it should be emphasized and noted that "an embodiment" or "one embodiment" or "an alternative embodiment" mentioned two or more times in different positions in the present disclosure does not necessarily refer to the same embodiment. Furthermore, certain features, structures, or characteristics in one or more embodiments of the present disclosure may be appropriately combined.

Furthermore, unless explicitly stated in the claims, the order of processing elements and sequences, the use of numbers and letters, or the use of other names in the present disclosure are not intended to limit the order of the processes and methods of the present disclosure. Although the above disclosure discusses some inventive embodiments currently considered useful through various examples, it should be understood that such details are for illustrative purposes only, and the appended claims are not limited to the disclosed embodiments. On the contrary, the claims are intended to cover all modifications and equivalent combinations that conform to the substance and scope of the embodiments of the present disclosure. For example, although the implementation of various components described above may be embodied in a hardware device, it may also be implemented as a software only solution, e.g., an installation on an existing server or mobile device.

Similarly, it should be noted that in order to simplify the expression disclosed in the present disclosure and thereby aiding in the understanding of one or more inventive embodiments, sometimes a plurality of features are grouped into one embodiment, drawing, or description thereof in the foregoing description of the embodiments of the present disclosure. However, this method of disclosure does not mean that the object of the present disclosure requires more features than those mentioned in the claims. Rather, claimed subject matter may lie in less than all features of a single foregoing disclosed embodiment.

In some embodiments, numbers describing the quantity of components or attributes are used. It should be understood that such numbers used in the description of the embodiments are modified by the modifiers "approximately," "approximate," or "substantially" in some embodiments. Unless otherwise stated, "approximately," "approximate," or "substantially" indicates that the stated number allows a variation of ±20%. Accordingly, in some embodiments, the numerical parameters used in the present disclosure and claims are approximate values, which varies according to the characteristics required by the individual embodiments. In some embodiments, the numerical parameters should consider the specified number of significant digits and adopt the method of general digit retention. Although the numerical ranges and parameters used to confirm the breadth of their scope in some embodiments of the present disclosure are approximate values, in specific embodiments, the setting of such numerical values is as precise as possible within the feasible range.

For each patent, patent application, patent application publication, and other material, such as articles, books, specifications, publications, documents, etc., cited in the present disclosure, the entire content thereof is hereby incorporated into the present disclosure by reference. Except for application history documents that are inconsistent with or conflict with the content of the present disclosure, and documents that limit the broadest scope of the claims of the present disclosure (currently or later appended to the present disclosure) are also excluded. It should be noted that if the description, definition, and/or use of terms in the ancillary materials of the present disclosure are inconsistent with or conflict with the content described in the present disclosure, the description, definition, and/or use of terms in the present disclosure shall prevail.

Finally, it should be understood that the embodiments described in the present disclosure are only used to illustrate the principles of the embodiments of the present disclosure. Other variations may also fall within the scope of the present disclosure. Therefore, by way of example and not limitation, alternative configurations of the embodiments of the present disclosure may be considered consistent with the teachings of the present disclosure. Accordingly, the embodiments of the present disclosure are not limited to the embodiments explicitly introduced and described in the present disclosure.

## Claims

1. A method for intelligent control, comprising:
obtaining a first voice signal, the first voice signal being generated by a first voice device through detecting a first user voice;
obtaining a voice recognition result by performing a voice recognition on the first voice signal;
determining, based on the voice recognition result, whether the first user voice contains a control instruction for a target device; and
in response to determining that the first user voice contains the control instruction for the target device, controlling the target device based on the control instruction.

2. The method of claim 1, wherein the target device includes a display device installed in an examination room, and the control instruction includes at least one of the following instructions:
an instruction for switching a display image on the display device;
an instruction for playing or pausing playing the display image on the display device;
an instruction for switching a display mode of the display device;
an instruction for setting a display parameter of the display image on the display device;
an instruction for processing the display image on the display device;
an instruction for setting a segmentation mode of a display region of the display device; and
an instruction for redirecting a display interface to a user configuration interface.

3. The method of claim 1 or 2, wherein the target device includes a medical device installed in the examination room, and the control instruction includes at least one of the following instructions:
an instruction for performing a motion control on a component of the medical device;
an instruction for making the medical device to perform a medical procedure on a target region of a target object;
an instruction for setting a working mode of the medical device;
an instruction for setting a relevant parameter of the medical procedure corresponding to the medical device; and
an instruction for making the medical device to start or stop the medical procedure.

4. The method of any one of claims 1-3, further comprising:
controlling the first voice device to play a feedback voice.

5. The method of claim 4, wherein the controlling the first voice device to play the feedback voice includes:
determining a security level of the control instruction based on the voice recognition result;
determining a content of the feedback voice based on the security level; and
controlling the first voice device to play the feedback voice based on the content of the feedback voice.

6. The method of claim 1, wherein before obtaining the first voice signal, the method further comprises:
obtaining a second voice signal, the second voice signal being generated by the first voice device through detecting a second user voice;
determining whether the second user voice contains a wake-up word; and
in response to determining that the second user voice contains the wake-up word, starting a voice control mode.

7. The method of any one of claims 1-5, wherein
the method further comprises: determining, based on the voice recognition result, whether the first user voice contains a wake-up word; and
the in response to determining that the first user voice contains the control instruction for the target device, the controlling the target device based on the control instruction includes: in response to determining that the first user voice contains the wake-up word and contains the control instruction for the target device, controlling the target device based on the control instruction.

8. The method of claim 3, wherein the medical device is a C-arm X-ray imaging device.

9. The method of any one of claims 1-8, wherein the obtaining a voice recognition result by performing the voice recognition on the first voice signal includes:
determining, based on the first voice signal, first voiceprint information of at least one user corresponding to the first voice signal;
determining, based on the first voiceprint information of the at least one user, whether the at least one user contains a target user;
in response to determining that the at least one user contains the target user, determining a target voice signal corresponding to the target user; and
obtaining the voice recognition result by performing the voice recognition on the target voice signal.

10. The method of claim 9, wherein the determining, based on the first voiceprint information of the at least one user, whether the at least one user contains the target user includes:
obtaining target voiceprint information corresponding to the target user; and
determining whether the at least one user contains the target user by comparing the first voiceprint information of the at least one user and the target voiceprint information.

11. The method of claim 9, wherein the determining, based on the first voiceprint information of the at least one user, whether the at least one user contains a target user includes:
for each of the at least one user,
determining identity information of the user based on the first voiceprint information;
determining a discourse weight of the user based on the identity information of the user; and
determining whether the user is the target user based on the discourse weight of the user.

12. The method of claim 6, wherein the target device includes a display device installed in an examination room, and before obtaining the first voice signal, the method further comprises:
determining second voiceprint information corresponding to the second user voice based on the second user voice; and
determining an initial display parameter of the display device based on the second voiceprint information.

13. The method of any one of claims 1-12, wherein the method further comprises:
determining whether the first user voice contains an inquiry based on the voice recognition result;
in response to determining that the first user voice contains the inquiry, determining response information corresponding to the inquiry; and
controlling the first voice device to play the response information.

14. The method of claim 1, wherein the target device includes a display device including a target display region configured to display feature information of a target object, and the method further comprises:
obtaining abnormal feature information of the target object;
updating a display mode of the target display region based on the abnormal feature information; and
controlling the first voice device or a wearable device worn by a target user to issue alert information.

15. The method of claim 3, wherein the control instruction is an instruction for performing a motion control on a component of the medical device, and the method further comprises:
during motion of the component, obtaining a first real-time optical image related to the medical device, the first real-time optical image being obtained by an image obtaining device installed in the examination room; and
determining whether a collision occurs during the motion of the component based on the first real-time optical image.

16. The method of claim 15, wherein the method further comprises:
before obtaining the first voice signal, obtaining a second real-time optical image related to the medical device obtained by the image obtaining device;
controlling the display device to display a target interface, the target interface being used to reflect a real-time status of the target object and the medical device; and
during the motion of the component, updating, based on the first real-time optical image, the target interface.

17. The method of claim 15, wherein the method further comprises:
in response to determining that the collision occurs during the motion of the component, controlling the first voice device to issue a collision warning;
obtaining a third voice signal, the third voice signal being generated by the first voice device through detecting a third user voice; and
determining a collision response strategy based on the third voice signal.

18. The method of claim 3, wherein the control instruction is the instruction for making the medical device to perform the medical procedure on the target region of the target object, and the method further comprises:
obtaining a third real-time optical image related to the medical device and the target object captured by the image obtaining device installed in the examination room;
determining planned motion information of a component of the medical device based on the third real-time optical image; and
controlling the component of the medical device to move based on the planned motion information to perform the medical procedure on the target region.

19. The method of claim 1, wherein the target device includes a medical device installed in an examination room, and the controlling the target device includes:
sending the control instruction to a system control device, so that the system control device controls the medical device.

20. The method of claim 19, wherein the sending the control instruction to a system control device, so that the system control device controls the medical device includes:
generating a structured control instruction by performing a structured processing on the control instruction, wherein the structured control instruction has a preset data structure; and
sending the structured control instruction to the system control device, so that the system control device controls the medical device.

21. The method of claim 1, wherein the target device includes a medical device and a display device installed in an examination room, and the controlling the target device based on the control instruction comprises:
in response to the control instruction, controlling the display device to display a medical procedure performed by the medical device.

22. The method of any one of claims 1-21, wherein the method further comprises:
sending the first voice signal to a second voice device installed in a control room, so that the second voice device plays the first user voice.

23. A system for intelligent control, comprising an obtaining module, a recognition module, a determination module, and a control module, wherein
the obtaining module is configured to obtain a first voice signal, the first voice signal being generated by a first voice device through detecting a first user voice;
the recognition module is configured to obtain a voice recognition result by performing a voice recognition on the first voice signal;
the determination module is configured to determine, based on the voice recognition result, whether the first user voice contains a control instruction for a target device; and
the control module is configured to, in response to determining that the first user voice contains the control instruction for the target device, control the target device based on the control instruction.

24. A system for intelligent control, comprising:
a storage device storing computer instructions;
a processor connected to the storage device, wherein when the computer instructions are executed, the processor makes the system to perform the following operations:
obtaining a first voice signal, the first voice signal being generated by a first voice device through detecting a first user voice;
obtaining a voice recognition result by performing a voice recognition on the first voice signal;
determining, based on the voice recognition result, whether the first user voice contains a control instruction for a target device; and
in response to determining that the first user voice contains the control instruction for the target device, controlling the target device based on the control instruction.

25. A computer-readable storage medium, the storage medium storing computer instructions, wherein when a computer reads the computer instructions from the storage medium, the computer executes the method of any one of claims 1-22.
